# EUROPEAN PATENT APPLICATION

(11) **EP 1 260 221 A2**
(43) Date of publication of application: **27.11.2002**
(21) Application number: 02253135.4
(22) Date of filing: 03.05.2002
(51) Int. Cl.: A61K 31/085, A61K 31/135

(54) **Combination treatment for depression and anxiety**

(30) Priority: 23.05.2001 US 293063
(71) Applicant: Pfizer Products Inc., Groton, Connecticut 06340 (US)
(72) Inventor: Howard, Harry Ralph, Jr., Groton, Connecticut 06340 (US)
(74) Representative: Stuart, Peter Graham

(57) **Abstract**

The present invention relates to a method of treating depression or anxiety in a mammal, including a human, by administering to the mammal a monoamine reuptake inhibitor in combination with a dopamine D3 receptor agonist. It also relates to pharmaceutical compositions containing a pharmaceutically acceptable carrier, a monoamine reuptake inhibitor and a dopamine D3 receptor agonist.

## Description

### Background Of The Invention

The present invention relates to a method of treating depression or anxiety in a mammal, including a human, by administering to the mammal a biaryl ether derivative, as described below, in combination with a dopamine D3 receptor agonist. This invention also relates to pharmaceutical compositions containing a pharmaceutically acceptable carrier, a biaryl ether derivative, as described below, and a dopamine D3 receptor agonist.

The biaryl ether derivatives referred to above that are employed in the methods and pharmaceutical compositions of this invention exhibit activity as monoamine (*e.g.*, serotonin, dopamine, norepinephrine) reuptake inhibitors. These biaryl ether derivatives are referred to in United States Patent Application No. 09/692,335, filed October 19, 2000, and in International Patent Application No. WO 00/50380, published August 31, 2000.

### Summary Of The Invention

The present invention relates to a pharmaceutical composition for the treatment of depression or anxiety in a mammal, including a human, comprising: (a) a dopamine D3 receptor agonist, or pharmaceutically acceptable salt thereof; (b) a compound of the formula I, as depicted and defined below, 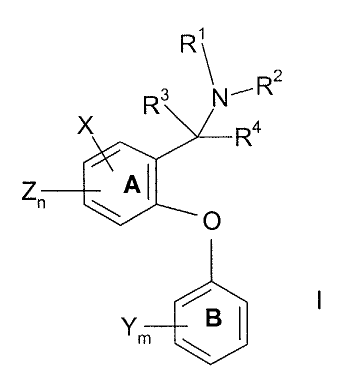 or pharmaceutically acceptable salt thereof, wherein phenyl ring A and phenyl ring B can each, independently, be replaced by a naphthyl group, and wherein when phenyl ring A is replaced by a naphthyl group, the ethereal oxygen of structure I and the carbon to which R³, R⁴ and NR¹R² are attached, are attached to adjacent ring carbon atoms of the naphthyl group and neither of said adjacent ring carbon atoms is also adjacent to a fused ring carbon atom of said naphthyl group;
n and m are selected, independently, from one, two and three;
R¹ and R² are selected, independently, from hydrogen, (C₁-C₄)alkyl, (C₂-C₄)alkenyl, and (C₂-C₄)alkynyl, or R¹ and R², together with the nitrogen to which they are attached, form a four to eight membered saturated ring containing one or two heteroatoms, including the nitrogen to which R¹ and R² are attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
R³ and R⁴ are selected, independently, from hydrogen and (C₁-C₄) alkyl optionally substituted with from one to three fluorine atoms, or R³ and R⁴ together with the carbon to which they are attached form a four to eight membered saturated carbocyclic ring, and wherein said ring may optionally be substituted at available binding sites with from one to three substituents selected, independently, from hydroxy and (C₁-C₆)alkyl;
or R² and R³, together with the nitrogen to which R² is attached and the carbon to which R³ is attached, form a four to eight membered saturated ring containing one or two heteroatoms, including the nitrogen to which R² is attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
each X is selected, independently, from phenyl, heteroaryl (*e.g.*, furan, thiophene, pyrrole, thiazole, isothiazole, oxazole, isoxazole, imidazole, 1,2,4-oxadiazole, 1,2,4-thiadiazole, 1,2,4-triazole, 1,2,3,-triazole, tetrazole, pyridine, pyrimidine, pyrazine, quinoline, isoquinoline, quinazoline, quinoxaline, benzothiophene, benzofuran, benzimidazole, benzisoxazole, benzisothiazole and indole) and heterocyclic (*e.g.*, imidazolidine, oxazolidine, thiazolidine, pyrrolidine, piperidine, morpholine) groups, as defined below, and may be further substituted by hydrogen, halo (*i*.*e*., fluorine, chlorine, bromine, iodine), (C₁-C₄)alkyl optionally substituted with from one to three fluorine atoms, (C₁-C₄)alkoxy optionally substituted with from one to three fluorine atoms, cyano, nitro, amino, hydroxy, carbonyl, (C₁-C₄)alkylamino, di-[(C₁-C₄)alkyl]amino, NR⁵(C=O)(C₁-C₄)alkyl, SO₂NR⁵R⁶ or SOₚ(C₁-C₆)alkyl, wherein R⁵ and R⁶ are selected, independently, from hydrogen and (C₁-C₆)alkyl, and p is zero, one or two;
each Y is selected, independently, from hydrogen, halo (*i*.*e*., chloro, fluoro, bromo or iodo), (C₁-C₄)alkyl optionally substituted with from one to three fluorine atoms, (C₁-C₄)alkoxy optionally substituted with from one to three fluorine atoms, cyano, nitro, amino, (C₁-C₄)alkylamino, di-[(C₁-C₄)alkyl]amino, NR⁵(C=O)(C₁-C₄)alkyl, SO₂NR⁵R⁶ and SOₚ(C₁-C₆)alkyl, wherein R⁵ and R⁶ are selected, independently, from hydrogen and (C₁-C₆)alkyl, and p is zero, one or two; and
each Z is selected independently from hydrogen, halo (*i*.*e*., chloro, fluoro, bromo or iodo), (C₁-C₄)alkyl optionally substituted with from one to three fluorine atoms, and (C₁-C₄)alkoxy; and (c) a pharmaceutically acceptable carrier; wherein the active agents "a" and "b" above are present in amounts that render the composition effective in treating, respectively, depression or anxiety.

This invention also relates to a method of treating depression or anxiety in a mammal, including a human, comprising administering to said mammal a pharmaceutical composition comprising: (a) a dopamine D3 receptor agonist, or pharmaceutically acceptable salt thereof; (b) a compound of the formula I, as defined above, or pharmaceutically acceptable salt thereof; and (c) a pharmaceutically acceptable carrier; wherein the active agents "a" and "b" above are present in amounts that render the composition effective in treating, respectively, depression or anxiety.

This invention also relates to a method of treating depression or anxiety in a mammal, including a human, comprising administering to said mammal: (a) a dopamine D3 receptor agonist, or pharmaceutically acceptable salt thereof; and (b) a compound of the formula I, as defined above, or pharmaceutically acceptable salt thereof; wherein the active agents "a" and "b" above are present in amounts that render the combination of the two agents effective in treating, respectively, depression or anxiety.

The present invention also relates to a pharmaceutical composition for the treatment of depression or anxiety in a mammal, including a human, comprising: (a) a dopamine D3 receptor agonist, or pharmaceutically acceptable salt thereof; (b) a compound of the formula XXX, as depicted and defined below, 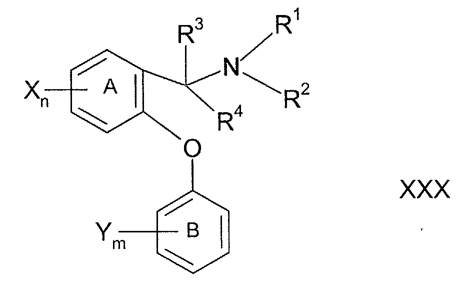 or pharmaceutically acceptable salt thereof, wherein phenyl ring A and phenyl ring B can each, independently, be replaced by a naphthyl group, and wherein when phenyl ring A is replaced by a naphthyl group, the ethereal oxygen of structure I and the carbon to which R³, R⁴ and NR¹R² are attached, are attached to adjacent ring carbon atoms of the naphthyl group and neither of said adjacent ring carbon atoms is also adjacent to a fused ring carbon atom of said naphthyl group;
n and m are, selected, independently, from one, two and three;
R¹ and R² are selected, independently, from hydrogen, (C₁-C₄)alkyl, (C₂-C₄)alkenyl, and (C₂-C₄)alkynyl, or R¹ and R², together with the nitrogen to which they are attached, form a four to eight membered saturated ring containing one or two heteroatoms, including the nitrogen to which R¹ and R² are attached, wherein the second heteroatom, when present, is selected from oxygen, nitrogen and sulfur, with the proviso that said ring can not contain two adjacent oxygen atoms or two adjacent sulfur atoms, and wherein said ring may optionally be substituted at available binding sites with from one to three substituents selected, independently, from hydroxy and (C₁-C₆)alkyl;
R³ and R⁴ are selected, independently, from hydrogen and (C₁-C₄) alkyl optionally substituted with from one to three fluorine atoms, or R³ and R⁴, together with the carbon to which they are attached, form a four to eight membered saturated carbocyclic ring, and wherein said ring may optionally be substituted at available binding sites with from one to three substituents selected, independently, from hydroxy and (C₁-C₆)alkyl;
or R² and R³, together with the nitrogen to which R² is attached and the carbon to which R³ is attached, form a four to eight membered saturated ring containing one or two heteroatoms, including the nitrogen to which R² is attached, wherein the second heteroatom, when present, is selected from oxygen, nitrogen and sulfur, with the proviso that said ring can not contain two adjacent oxygen atoms or two adjacent sulfur atoms, and wherein said ring may optionally be substituted at available binding sites with from one to three substituents selected, independently, from hydroxy and (C₁-C₆)alkyl;
each X is selected, independently, from hydrogen, halo *(i.e.,* chloro, fluoro, bromo or iodo), (C₁-C₄)alkyl optionally substituted with from one to three fluorine atoms, (C₁-C₄)alkoxy optionally substituted with from one to three fluorine atoms, cyano, nitro, amino, (C₁-C₄)alkylamino, di-[(C₁-C₄)alkyl]amino, NR⁵(C=O)(C₁-C₄)alkyl, SO₂NR⁵R⁶ and SOₚ(C₁-C₆)alkyl, wherein R⁵ and R⁶ are selected, independently, from hydrogen and (C₁-C₆)alkyl, and p is zero, one or two; and
each Y is selected, independently, from hydrogen, (C₁-C₆)alkyl and halo; with the proviso that: (1) no more than one of NR¹R², CR³R⁴ and R²NCR³ can form a ring; and (2) at least one X must be other than hydrogen when (i) R³ and R⁴ are both hydrogen, (ii) R¹ and R² are selected, independently, from hydrogen and (C₁-C₄)alkyl, and (iii) ring B is mono- or disubstituted with, respectively, one or two halo groups; and (c) a pharmaceutically acceptable carrier; wherein the active agents "a" and "b" above are present in amounts that render the composition effective in treating, respectively, depression or anxiety.

This invention also relates to a method of treating depression or anxiety in a mammal, including a human, comprising administering to said mammal a pharmaceutical composition comprising: (a) a dopamine D3 receptor agonist, or pharmaceutically acceptable salt thereof; (b) a compound of the formula XXX, as defined above, or pharmaceutically acceptable salt thereof; and (c) a pharmaceutically acceptable carrier; wherein the active agents "a" and "b" above are present in amounts that render the composition effective in treating, respectively, depression or anxiety.

This invention also relates to a method of treating depression or anxiety in a mammal, including a human, comprising administering to said mammal: (a) a dopamine D3 receptor agonist, or pharmaceutically acceptable salt thereof; and (b) a compound of the formula XXX, as defined above, or pharmaceutically acceptable salt thereof; wherein the active agents "a" and "b" above are present in amounts that render the combination of the two agents effective in treating, respectively, depression or anxiety.

The present invention also relates to a pharmaceutical composition for the treatment of depression or anxiety in a mammal, including a human, comprising: (a) a dopamine D3 receptor agonist, or pharmaceutically acceptable salt thereof; (b) a compound of the formula XXXI or XXXII, respectively, as depicted below, 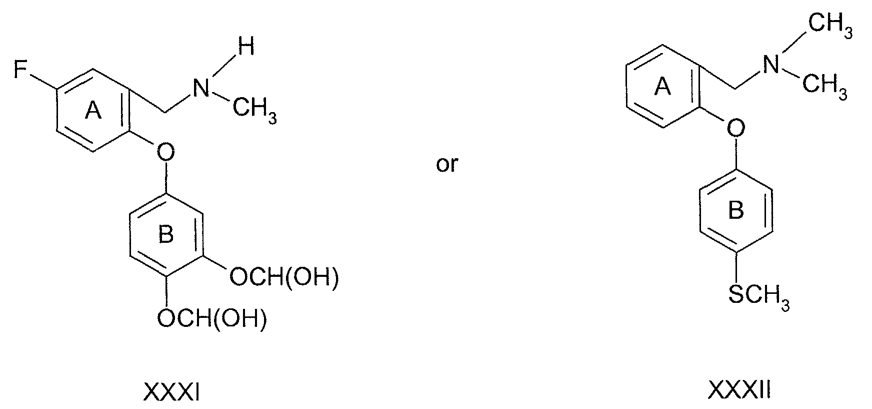 or pharmaceutically acceptable salt thereof; and (c) a pharmaceutically acceptable carrier; wherein the active agents "a" and "b" above are present in amounts that render the composition effective in treating, respectively, depression or anxiety.

This invention also relates to a method of treating depression or anxiety in a mammal, including a human, comprising administering to said mammal a pharmaceutical composition comprising: (a) a dopamine D3 receptor agonist, or pharmaceutically acceptable salt thereof; (b) a compound of the formula XXXI or XXXII, respectively, as defined above, or pharmaceutically acceptable salt thereof; and (c) a pharmaceutically acceptable carrier; wherein the active agents "a" and "b" above are present in amounts that render the composition effective in treating, respectively, depression or anxiety.

This invention also relates to a method of treating depression or anxiety in a mammal, including a human, comprising administering to said mammal: (a) a dopamine D3 receptor agonist, or pharmaceutically acceptable salt thereof; and (b) a compound of the formula XXXI or XXXII, respectively, as defined above, or pharmaceutically acceptable salt thereof; wherein the active agents "a" and "b" above are present in amounts that render the combination of the two agents effective in treating, respectively, depression or anxiety.

It will be appreciated that when using a combination method of the present invention, referred to immediately above, both the monoamine reuptake inhibitor (*i*.*e*., the compound of formula I, XXX, XXXI or XXXII) and the dopamine D3 receptor agonist will be administered to a patient within a reasonable period of time. The compounds may be in the same pharmaceutically acceptable carrier and therefore administered simultaneously. Alternatively, they may be in separate pharmaceutical carriers such as conventional oral dosage forms that are administered simultaneously or within a time period that one of skill in the art would consider reasonable for effecting the desired combination therapy sequentially. By way of example, the dopamine D3 receptor agonist may be administered as a tablet and then, within a reasonable period of time, the monoamine reuptake inhibitor (*i*.*e*., the compound of formula I, XXX, XXXI or XXXII) may be administered either as a tablet or a fast-dissolving oral dosage form.

The compositions of the present invention that contain a monoamine reuptake inhibitor (*i*.*e*., a compound of formula I, XXX, XXXI or XXXII) and a dopamine D3 receptor agonist are useful for the treatment of depression. As used herein, the term "depression" includes depressive disorders, for example, single episodic or recurrent major depressive disorders, dysthymic disorders, cyclothymic disorder, depressive neurosis, and neurotic depression; melancholic depression including anorexia, weight loss, insomnia and early morning waking, and psychomotor retardation; atypical depression (or reactive depression) including increased appetite, hypersomnia, psychomotor agitation or irritability, anxiety and phobias. The term "depression," as used herein, also includes the mood disorders such as mood disorders associated with premenstrual syndrome (PMS) or premenstrual dysphoric disorder (PMDD), seasonal affective disorder and bipolar disorders or manic depression, for example, bipolar I disorder and bipolar II disorder.

Major depression is characterized by feelings of intense sadness and despair, mental slowing and loss of concentration, pessimistic worry, agitation, and self-deprecation. Physical changes also occur, especially in severe or "melancholic" depression. These include insomnia or hypersomnia, anorexia and weight loss (or sometimes overeating), decreased energy and libido, and disruption of normal circadian rhythms of activity, body temperature, and many endocrine functions.

Treatment regimens commonly include the use of tricyclic antidepressants, monoamine oxidase inhibitors, some psychotropic drugs, lithium carbonate, and electroconvulsive therapy (ECT) (see R. J. Baldessarini in *Goodman & Gilman's The Pharmacological Basis of Therapeutics,* 9th Edition, Chapter 19, McGraw-Hill, 1996 for a review). More recently, new classes of antidepressant drugs have been or are being developed including selective serotonin reuptake inhibitors (SSRIs), specific monoamine reuptake inhibitors and 5-HT_{IA/ID} receptor agonists, antagonists and partial agonists.

Selective serotonin reuptake inhibitors (SSRIs) currently provide efficacy in the treatment of major depressive disorder (MDD) and are generally perceived by psychiatrists and primary care physicians as effective, well-tolerated and easily administered. However, they are associated with undesirable features, such as reports of sexual dysfunction, delayed onset of action and a level of non-responsiveness estimated to be as high as 30% (see M. J. Gitlin, Journal of Clinical Psychiatry, 1994, 55, 406-413 and R. T. Segraves, Journal of Clinical Psychiatry, 1992, 10(2), 4-10). Preclinical and clinical evidence has indicated that the sexual dysfunction associated with SSRI therapy can be reduced through the use of dopamine reuptake inhibitors (DRIs), such as bupropion (see A. K. Ashton, Journal of Clinical Psychiatry, 1998, 59(3), 112-115). Furthermore, the combination of SRI and DRI may hasten the onset of action as well as offering relief to refractory patients, possibly through a synergistic mechanism (see R. D. Marshall et al, Journal of Psychopharmacology, 1995, 9(3), 284-286).

Other mood disorders encompassed within the term "depression" include dysthymic disorder with early or late onset and with or without atypical features; dementia of the Alzheimer's type, with early or late onset, with depressed mood; vascular dementia with depressed mood; mood disorders induced by alcohol, amphetamines, cocaine, hallucinogens, inhalants, opioids, phencyclidine, sedatives, hypnotics, anxiolytics and other substances; schizoaffective disorder of the depressed type; and adjustment disorder with depressed mood.

The compositions of the present invention that contain a monoamine reuptake inhibitor (*i*.*e*., a compound of formula I, XXX, XXXI or XXXII) and a dopamine D3 receptor agonist are useful for the treatment of anxiety. As used herein, the term "anxiety" includes anxiety disorders, such as panic disorder with or without agoraphobia, agoraphobia without history of panic disorder, specific phobias, for example, specific animal phobias, social phobias, obsessive-compulsive disorder, stress disorders including post-traumatic stress disorder and acute stress disorder, and generalized anxiety disorders.

Anxiety disorders are generally treated using benzodiazepine sedative-antianxiety agents. Potent benzodiazepines are effective in panic disorder as well as in generalized anxiety disorder, however, the risks associated with drug dependency may limit their long-term use. 5-HT_{1A} receptor partial agonists also have useful anxiolytic and other psychotropic activity, and less likelihood of sedation and dependence (see R. J. Baldessarini in *Goodman &* *Gilman's The Pharmacological Basis of Therapeutics,* 9th Edition, Chapter 18, McGraw-Hill, 1996 for a review).

"Generalized anxiety" is typically defined as an extended period (*e.g.*, at least six months) of excessive anxiety or worry with symptoms on most days of that period. The anxiety and worry is difficult to control and may be accompanied by restlessness, being easily fatigued, difficulty concentrating, irritability, muscle tension, and disturbed sleep.

"Panic disorder" is defined as the presence of recurrent panic attacks followed by at least one month of persistent concern about having another panic attack. A "panic attack" is a discrete period in which there is a sudden onset of intense apprehension, fearfulness or terror. During a panic attack, the individual may experience a variety of symptoms including palpitations, sweating, trembling, shortness of breath, chest pain, nausea and dizziness. Panic disorder may occur with or without agoraphobia.

"Phobias" includes agoraphobia, specific phobias and social phobias. "Agoraphobia" is characterized by an anxiety about being in places or situations from which escape might be difficult or embarrassing or in which help may not be available in the event of a panic attack. Agoraphobia may occur without history of a panic attack. A "specific phobia" is characterized by clinically significant anxiety provoked by a feared object or situation. Specific phobias include the following subtypes: animal type, cued by animals or insects; natural environment type, cued by objects in the natural environment, for example storms, heights or water; blood-injection-injury type, cued by the sight of blood or an injury or by seeing or receiving an injection or other invasive medical procedure; situational type, cued by a specific situation such as public transportation, tunnels, bridges, elevators, flying, driving or enclosed spaces; and other types where fear is cued by other stimuli. Specific phobias may also be referred to as simple phobias. A "social phobia" is characterized by clinically significant anxiety provoked by exposure to certain types of social or performance circumstances. Social phobia may also be referred to as social anxiety disorder.

Other anxiety disorders encompassed within the term "anxiety" include anxiety disorders induced by alcohol, amphetamines, caffeine, cannabis, cocaine, hallucinogens, inhalants, phencyclidine, sedatives, hypnotics, anxiolytics and other substances, and adjustment disorders with anxiety or with mixed anxiety and depression.

Anxiety may be present with or without other disorders such as depression in mixed anxiety and depressive disorders. The compositions of the present invention are useful in the treatment of anxiety with or without accompanying depression. They are also useful in the treatment of depression with or without accompanying anxiety.

The compositions of the present invention are especially useful for the treatment of depression or anxiety where the use of an antidepressant or anxiolytic agent, respectively, is generally prescribed. By the use of a combination of a monoamine reuptake inhibitor (*i*.*e*., a compound of formula I, XXX, XXXI or XXXII) and a dopamine D3 receptor agonist in accordance with the present invention, it is possible to treat depression and/or anxiety in patients for whom conventional antidepressant or antianxiety therapy might not be wholly successful or where dependence upon the antidepressant or antianxiety therapy is prevalent.

Unless otherwise indicated, the term "halo", as used in the compounds of formula I and XXX, respectively, includes fluoro, chloro, bromo and iodo.

Unless otherwise indicated, the term "alkyl", as used in the compounds of formula I and XXX, respectively, may be straight, branched or cyclic, and may include straight and cyclic moieties as well as branched and cyclic moieties.

Unless otherwise indicated, the term "alkenyl", as used in the compounds of formula I and XXX, respectively, includes unsaturated hydrocarbon radicals having one or more double bonds connecting two carbon atoms, wherein said hydrocarbon radical may have straight, branched or cyclic moieties or combinations thereof. Examples of "alkenyl" groups include, but are not limited to, ethenyl, propenyl, butenyl, pentenyl and dimethylpentyl, and include E and Z forms where applicable.

Unless otherwise indicated, the term "alkynyl", as used in the compounds of formula I and XXX, respectively, includes unsaturated hydrocarbon radicals having one or more triple bonds connecting two carbon atoms, wherein said hydrocarbon radical may have straight, branched or cyclic moieties or combinations thereof.

Unless otherwise indicated, the term "heteroaryl", as used in the compounds of formula I, refers to aromatic groups containing one or more heteroatoms (O, S, or N), preferably from one to four heteroatoms. Unless otherwise indicated, a multicyclic group containing one or more heteroatoms wherein at least one ring of the group is aromatic is also a "heteroaryl" group for purposes of the present invention. The heteroaryl groups of the compounds of this invention can also include ring systems substituted with one or more oxo moieties. Examples of heteroaryl groups are pyridinyl, pyridazinyl, imidazolyl, pyrimidinyl, pyrazolyl, triazolyl, pyrazinyl, quinolyl, isoquinolyl, tetrazolyl, furyl, thiophenyl, isoxazolyl, thiazolyl, oxazolyl, isothiazolyl, pyrrolyl, indolyl, benzimidazolyl, benzofuranyl, cinnolinyl, indazolyl, indolizinyl, phthalazinyl, triazinyl, isoindolyl, purinyl, oxadiazolyl, thiadiazolyl, furazanyl, benzofurazanyl, benzothiophenyl, benzotriazolyl, benzothiazolyl, benzisothiazolyl, benzoxazolyl, benzisoxazolyl, benzimidazolyl, quinazolinyl, quinoxalinyl, naphthyridinyl, dihydroquinolyl, tetrahydroquinolyl, dihydroisoquinolyl, tetrahydroisoquinolyl, benzofuryl, furopyridinyl, pyrolopyrimidinyl and azaindolyl.

Unless otherwise indicated, the term "heterocyclic", as used in the compounds of formula I, refers to non-aromatic cyclic groups containing one or more heteroatoms, preferably from one to four heteroatoms, each selected from O, S and N. Unless otherwise indicated, "heterocyclic" includes heterobicyclic groups. "Heterobicyclic" refers to non-aromatic two-ringed cyclic groups, wherein said rings share one or two atoms, and wherein at least one of the rings contains a heteroatom (O, S, or N). Unless otherwise indicated, for purposes of the present invention, heterobicyclic groups include spiro groups and fused ring groups. In one embodiment, each ring in the heterobicyclic group contains up to four heteroatoms (*i*.*e*., from zero to four heteroatoms, provided that at least one ring contains at least one heteroatom). The heterocyclic groups of this invention can also include ring systems substituted with one or more oxo moieties. Examples of heterocyclic groups include, but are not limited to, aziridinyl, azetidinyl, pyrrolidinyl, piperidinyl, azepinyl, piperazinyl, 1,2,3,6-tetrahydropyridinyl, oxiranyl, oxetanyl, tetrahydrofuranyl, tetrahydrothienyl, tetrahydropyranyl, tetrahydrothiopyranyl, oxazolidinyl, morpholino, thiomorpholino, thiazolidinyl, thioxanyl, pyrrolinyl, indolinyl, 2H-pyranyl, 4H-pyranyl, dioxanyl, 1,3-dioxolanyl, pyrazolinyl, dihydropyranyl, dihydrothienyl, dihydrofuranyl, pyrazolidinyl, imidazolinyl, imidazolidinyl, 3-azabicyclo[3.1.0]hexanyl, 3-azabicyclo[4.1.0]heptanyl, quinolizinyl, quinuclidinyl, 1,4-dioxaspiro[4.5]decyl, 1,4-dioxaspiro[4.4]nonyl, 1,4-dioxaspiro[4.3]octyl and 1,4-dioxaspiro[4.2]heptyl.

The foregoing groups, heteroaryl or heterocyclic, may be C-attached or N-attached where such is possible. For instance, a group derived from pyrrole may be pyrrol-1-yl (N-attached) or pyrrol-3-yl (C-attached). The terms referring to the groups also encompass all possible tautomers.

When reference is made to SOₚ(C₁-C₆)alkyl, and p is two, this indicates a sulfone, in other words, S(=O)₂(C₁-C₆)alkyl.

In certain embodiments, this invention relates to the above pharmaceutical compositions for the treatment of depression or anxiety, and the above methods of treating depression or anxiety, wherein the compound of formula I, or pharmaceutically acceptable salt thereof, is a compound wherein ring B is phenyl, not replaced with a naphthyl group.

In other embodiments, this invention relates to the above pharmaceutical compositions for the treatment of depression or anxiety, and the above methods of treating depression or anxiety, wherein the compound of formula I, or pharmaceutically acceptable salt thereof, is a compound wherein phenyl ring B is replaced with a naphthyl group.

In preferred embodiments, this invention relates to the above pharmaceutical compositions for the treatment of depression or anxiety, and the above methods of treating depression or anxiety, wherein the compound of formula I, or pharmaceutically acceptable salt thereof, is a compound wherein, when ring B is phenyl, each Y is hydrogen or halo.

In more preferred embodiments, this invention relates to the above pharmaceutical compositions for the treatment of depression or anxiety, and the above methods of treating depression or anxiety, wherein the compound of formula I, or pharmaceutically acceptable salt thereof, is a compound wherein m is 1 or 2, and each Y is chlorine.

In other embodiments, this invention relates to the above pharmaceutical compositions for the treatment of depression or anxiety, and the above methods of treating depression or anxiety, wherein the compound of formula I, or pharmaceutically acceptable salt thereof, is as described above, but wherein X is selected from furan, thiophene, pyrrole, and 1,2,3-triazole, and wherein X may be further substituted as recited above.

In other embodiments, this invention relates to the above pharmaceutical compositions for the treatment of depression or anxiety, and the above methods of treating depression or anxiety, wherein the compound of formula I, or pharmaceutically acceptable salt thereof, is a compound wherein X is a lactam, for example 1-pyrrolidin-2-one or 1-piperidin-2-one, optionally substituted as recited above and attached to ring A through the lactam nitrogen.

In other embodiments, this invention relates to the above pharmaceutical compositions for the treatment of depression or anxiety, and the above methods of treating depression or anxiety, wherein the compound of formula I, or pharmaceutically acceptable salt thereof, is a compound wherein X is a tetrazole optionally substituted as recited above and attached to ring A through the tetrazole carbon.

In other embodiments, this invention relates to the above pharmaceutical compositions for the treatment of depression or anxiety, and the above methods of treating depression or anxiety, wherein the compound of formula I, or pharmaceutically acceptable salt thereof, is a compound as described above, but wherein each Z is selected from hydrogen and halo.

In other preferred embodiments, this invention relates to the above pharmaceutical compositions for the treatment of depression or anxiety, and the above methods of treating depression or anxiety, wherein the compound of formula I, or pharmaceutically acceptable salt thereof, is a compound as described above, but wherein Z is hydrogen.

In other embodiments, this invention relates to the above pharmaceutical compositions for the treatment of depression or anxiety, and the above methods of treating depression or anxiety, wherein the compound of formula I, or pharmaceutically acceptable salt thereof, is a compound as described above, wherein R³ and R⁴ are independently selected from hydrogen and unsubstituted (C₁-C₄) alkyl.

In other preferred embodiments, this invention relates to the above pharmaceutical compositions for the treatment of depression or anxiety, and the above methods of treating depression or anxiety, wherein the compound of formula I, or pharmaceutically acceptable salt thereof, is a compound wherein one or both of R³ and R⁴ are hydrogen.

In other embodiments, this invention relates to the above pharmaceutical compositions for the treatment of depression or anxiety, and the above methods of treating depression or anxiety, wherein the compound of formula I, or pharmaceutically acceptable salt thereof, is a compound wherein R¹ and R² are independently selected from hydrogen and unsubstituted (C₁-C₄)alkyl.

In other preferred embodiments, this invention relates to the above pharmaceutical compositions for the treatment of depression or anxiety, and the above methods of treating depression or anxiety, wherein the compound of formula I, or pharmaceutically acceptable salt thereof, is a compound wherein one of R¹ and R² is hydrogen and the other of R¹ and R² is (C₁-C₄)alkyl.

In more preferred embodiments, this invention relates to the above pharmaceutical compositions for the treatment of depression or anxiety, and the above methods of treating depression or anxiety, wherein the compound of formula I, or pharmaceutically acceptable salt thereof, is a compound wherein one of R¹ and R² is hydrogen and the other of R¹ and R² is methyl.

Other preferred embodiments of this invention relate to the above pharmaceutical compositions for the treatment of depression or anxiety, and the above methods of treating depression or anxiety, wherein the compound of formula I, or pharmaceutically acceptable salt thereof, is selected from the following compounds and their pharmaceutically acceptable salts:
[4-(3,4-Dichlorophenoxy)-biphenyl-3-ylmethyl]-methylamine;
[2-(3,4-Dichlorophenoxy)-5-thiophen-3-ylbenzyl]-methylamine;
[2-(3,4-Dichlorophenoxy)-4-thiophen-3-ylbenzyl]-methylamine;
[2-(3,4-Dichlorophenoxy)-4-furan-2-ylbenzyl]-methylamine;
[2-(3,4-Dichlorophenoxy)-5-furan-2-ylbenzyl]-methylamine;
N-[4'-(3,4-Dichlorphenoxy)-3'-methylaminomethyl-biphenyl-3-yl]-acetamide;
[2-(3,4-Dichlorophenoxy)-5-thiophen-2-ylbenzyl]-methylamine;
[4-(3,4-Dichlorophenoxy)-4'-fluoro-biphenyl-3-ylmethyl]-methyamine;
[2-(3,4-Dichlorophenoxy)-5-[1,2,3]triazol-1-ylbenzyl]-methylamine;
[2-(3,4-Dichlorophenoxy)-5-[1,2,3]triazol-2-ylbenzyl]-methylamine;
[2-(3,4-Dichlorophenoxy)-5-pyridin-2-ylbenzyl]-methylamine;
[2-(3,4-Dichlorophenoxy)-5-pyridin-3-ylbenzyl)-methylamine;
1-[4-(3,4-Dichlorophenoxy)-3-methylaminomethylphenyl]-1H-pyrazol-3-ylamine;
[2-(3,4-Dichlorophenoxy)-5-pyridin-4-ylbenzyl]-methylamine;
[3-(3,4-Dichlorophenoxy)-biphenyl-4-ylmethyl]-methylamine;
[4-(3,4-Dichlorophenoxy)-4'-methyl-biphenyl-3-ylmethyl]-methylamine; and
[2-(3,4-Dichlorophenoxy)-4-thiophen-2-ylbenzyl]-methylamine.

Additional preferred embodiments of this invention relate to the above pharmaceutical compositions for the treatment of depression or anxiety, and the above methods of treating depression or anxiety, wherein the compound of formula I, or pharmaceutically acceptable salt thereof, is selected from the following compounds and their pharmaceutically acceptable salts:
[2-(3,4-dichlorophenoxy)-5-thiazol-2-ylbenzyl]-methylamine;
[2-(3,4-dichlorophenoxy)-5-(1H-tetrazol-5-yl)benzyl]-methylamine;
[2-(3,4-dichlorophenoxy)-5-furan-3-ylbenzyl]-methylamine;
{1-[2-(3,4-dichlorophenoxy)-5-[1,2,3]triazol-1 -ylphenyl]ethyl}-methylamine;
{1-[2-(3,4-dichlorophenoxy)-5-[1,2,3]triazol-2-ylphenyl]ethyl}-methylamine;
{1-[2-(3,4-dichlorophenoxy)-5-thiazol-2-ylphenyl]ethyl}-methylamine;
{1-[2-(3,4-dichlorophenoxy)-4-[1,2,4]triazol-1-ylphenyl]ethyl}-methylamine;
[2-(3,4-dichlorophenoxy)-5-(5-methylthiophen-2-yl)benzyl]-methylamine;
[2-(3,4-dichlorophenoxy)-5-[1,2,4]triazol-4-ylbenzyl]-methylamine;
1-[4-(3,4-dichlorophenoxy)-3-(methylaminomethyl)phenyl]-pyrrolidin-2-one;
1-[4-(3,4-dichlorophenoxy)-3-(1-methylaminoethyl)phenyl]-pyrrolidin-2-one; and
1-[4-(3,4-dichlorophenoxy)-3-(methylaminomethyl)phenyl]-piperidin-2-one.

Other embodiments of this invention relate to the above pharmaceutical compositions for the treatment of depression or anxiety, and the above methods of treating depression or anxiety, wherein the compound of formula I, or pharmaceutically acceptable salt thereof, is selected from the following compounds and their pharmaceutically acceptable salts:
[2-(3,4-dichlorophenoxy)-5-pyrimidin-2-ylbenzyl]-methylamine;
[2-(3,4-dichlorophenoxy)-5-pyrimidin-4-ylbenzyl]-methylamine;
[2-(3,4-dichlorophenoxy)-5-(2-methylpyrimidin-4-yl)-benzyl]-methylamine;
{1-[2-(3,4-dichlorophenoxy)-5-(2-methylpyrimidin-4-yl)-phenyl]-ethyl}-methylamine;
4-[4-(3,4-dichlorophenoxy)-3-(1-methylpyrrolidin-2-yl)-phenyl]-2-methylpyrimidine;
[2-(4-chlorophenoxy)-5-(1-methyl-1H-pyrrol-3-yl)-benzyl]-dimethylamine;
[5-(1-methyl-1H-pyrrol-3-yl)-2-(naphthalen-2-yloxy)-benzyl]-dimethyl amine;
[5-imidazol-1-yl-2-(naphthalen-2-yloxy)-benzyl]-dimethylamine;
1,5,5-trimethyl-3-[3-methylaminomethyl-4-(naphthalen-2-yloxy)-phenyl]-imidazolidine-2,4-dione;
1-methyl-3-[3-methylaminomethyl-4-(naphthalen-2-yloxy)-phenyl]-imidazolidine-2,4-dione;
3-[3-methylaminomethyl-4-(naphthalen-2-yloxy)-phenyl]-thiazolidine-2,4-dione;
3-[3-methylaminomethyl-4-(naphthalen-2-yloxy)-phenyl]-oxazolidine-2,4-dione;
3-[3-methylaminomethyl-4-(naphthalen-2-yloxy)-phenyl]-oxazolidin-2-one;
3-[3-methylaminomethyl-4-(naphthalen-2-yloxy)-phenyl]-thiazolidin-2-one;
1-methyl-3-[3-methylaminomethyl-4-(naphthalen-2-yloxy)-phenyl]-imidazolidin-2-one;
1-methyl-3-[3-methylaminomethyl-4-(naphthalen-2-yloxy)-phenyl]-tetrahydro-pyrimidin-2-one;
1-[4-(3,4-dichlorophenoxy)-3-methylaminomethyl-phenyl]-3-methyl-tetrahydropyrimidin-2-one;
1-[4-(3,4-dichlorophenoxy)-3-methylaminomethyl-phenyl]-3-methylimidazolidin-2-one;
3-[4-(3,4-dichlorophenoxy)-3-methylaminomethyl-phenyl]-thiazolidin-2-one;
3-[4-(3,4-dichlorophenoxy)-3-methylaminomethyl-phenyl]-oxazolidin-2-one;
[2-(3,4-dichlorophenoxy)-5-(2-methylthiazol-4-yl)-benzyl]-methylamine;
[2-(3,4-dichlorophenoxy)-5-(2-methyloxazol-4-yl)-benzyl]-methylamine;
[2-(3,4-dichlorophenoxy)-5-(2,5-dimethyloxazol-4-yl)-benzyl]-methylamine;
[2-(3,4-dichlorophenoxy)-5-(2,5-dimethylthiazol-4-yl)-benzyl]-methylamine;
[2-(3,4-dichlorophenoxy)-5-(5-methyl-[1,2,4]thiadiazol-3-yl)-benzyl]-methylamine;
[2-(3,4-dichlorophenoxy)-5-(5-methyl-[1,2,4]oxadiazol-3-yl)-benzyl]-methylamine;
[2-(3,4-dichlorophenoxy)-5-[1,2,3]oxadiazol-4-yl-benzyl]-methylamine;
[2-(3,4-dichlorophenoxy)-5-(5-methyl-[1,2,3]thiadiazol-4-yl)-benzyl]-methylamine;
[2-(3,4-dichlorophenoxy)-5-(2,4-dimethyloxazol-5-yl)-benzyl]-methylamine;
[2-(3,4-dichlorophenoxy)-5-(2,4-dimethylthiazol-5-yl)-benzyl]-methylamine;
[2-(3,4-dichlorophenoxy)-5-[1,2,4]triazol-1-ylbenzyl]-methylamine;
[2-(3,4-dichlorophenoxy)-5-(3-methyl-[1,2,4]triazol-1-yl)-benzyl]-methylamine;
[2-(4-chlorophenoxy)-5-(3,5-dimethyl-[1,2,4]triazol-1-yl)-benzyl]-methylamine;
[2-(4-chlorophenoxy)-5-tetrazol-1-ylbenzyl]-methylamine;
[2-(4-chlorophenoxy)-5-(5-methyltetrazol-1-yl)-benzyl]-dimethylamine;
[2-(4-chlorophenoxy)-5-[12,4]triazol-4-ylbenzyl]-dimethylamine;
[2-(4-chlorophenoxy)-5-(1 -methyl-1H-tetrazol-5-yl)-benzyl]-dimethylamine; and
{1-[2-(3,4-dichlorophenoxy)-5-(1-methyl-1H-tetrazol-5-yl)-phenyl]-ethyl}-dimethylamine.

Other preferred embodiments of the present invention relate to the above pharmaceutical compositions for the treatment of depression or anxiety, and the above methods of treating depression or anxiety, wherein the compound of formula XXX, or pharmaceutically acceptable salt thereof, is selected from the following compounds and their pharmaceutically acceptable salts:
[2-(3,4-Dichlorophenoxy)-5-fluorobenzyl]-dimethylamine;
[2-(3,4-Dichlorophenoxy)-5-fluorobenzyl]-methylamine;
[2-(3,4-Dichlorophenoxy)-5-trifluoromethylbenzyl]-dimethylamine;
N-[4-(3,4-Dichlorophenoxy)-3-dimethylaminomethylphenyl]-acetamide;
{1-[2-(3,4-Dichlorophenoxy)phenyl]-ethyl}-dimethylamine;
[2-(3,4-Dichlorophenoxy)-4-trifluoromethylbenzyl]-dimethylamine;
[2-(3,4-Dichlorophenoxy)-4-trifluoromethylbenzyl]-methylamine;
[4-Chloro-2-(3,4-dichlorophenoxy)-benzyl]-methylamine;
{1-[2-(3,4-Dichlorophenoxy)-5-fluorophenyl]-ethyl}-methylamine;
{1-[2-(3,4-Dichlorophenoxy)phenyl}-ethyl}-methylamine;
{1-[2-(4-Chlorophenoxy)phenyl]ethyl}-methylamine;
[2-(3,4-Dichlorophenoxy)-5-methoxybenzyl]-methylamine;
[2-(4-Chlorophenoxy)-5-fluorobenzyl]-methylamine;
{1-[2-(4-Chlorophenoxy)-5-fluorophenyl]-ethyl}-methylamine;
[2-(3,4-Dichlorophenoxy)-5-methylbenzyl]-dimethylamine;
[4-Bromo-2-(3,4-dichlorophenoxy)-benzyl]-methylamine;
[5-Bromo-2-(3,4-dichlorophenoxy)-benzyl]-methylamine;
[2-(3,4-Dichlorophenoxy)-4,5-dimethoxybenzyl]-methylamine;
[2-(3,4-Dichlorophenoxy)-4-methoxybenzyl]-dimethylamine;
4-(3,4-Dichlorophenoxy)-3-methylaminomethyl-benzonitrile;
[2-(3,4-Dichlorophenoxy)-4,5-dimethylbenzyl]-methylamine;
3-(3,4-Dichlorphenoxy)-4-methylaminomethyl-benzonitrile;
(+)-{1-[2-(3,4-Dichlorophenoxy)-5-fluorophenyl]-ethyl}-methylamine;
(-)-{1-[2-(3,4-Dichlorophenoxy)-5-fluorophenyl]-ethyl}-methylamine;
[2-(3,4-Dichlorophenoxy)-5-trifluoromethyl-benzyl]-methylamine;
[2-(3,4-Dichlorophenoxy)-4-methoxybenzyl]-methylamine;
[2-(4-Chloro-3-fluorophenoxy)-5-fluorobenzyl]-methylamine;
[2-(3-Chloro-4-fluorophenoxy)-5-fluorobenzyl]-methylamine;
(+/-)-2-[2-(3,4-Dichlorophenoxy)-5-fluorophenyl]-pyrrolidine;
(-)-2-[2-(3,4-Dichlorophenoxy)-5-fluorophenyl]-pyrrolidine;
(+)-2-[2-(3,4-Dichlorophenoxy)-5-fluorophenyl]-pyrrolidine; and
2-[2-(3,4-Dichlorophenoxy)-5-fluorophenyl]-N-methylpyrrolidine.

Other embodiments of this invention relate to the above pharmaceutical compositions for the treatment of depression or anxiety, and the above methods of treating depression or anxiety, wherein the compound of formula XXX, or pharmaceutically acceptable salt thereof, is selected from the following compounds and their pharmaceutically acceptable salts:
{1-[2-(3,4-Dichlorophenoxy)-5-fluorophenyl]-1-methylethyl}-methylamine;
{1-[2-(3,4-Dichlorophenoxy)-5-fluorophenyl]-1-methylethyl}-dimethylamine;
[4-Chloro-2-(4-chlorophenoxy)-5-fluorobenzyl]-methylamine;
[2-(3,4-Dichlorophenoxy)-5-fluoro-4-methoxybenzyl]-methylamine;
[4-(3,4-Dichlorophenoxy)-3-(dimethylaminomethyl)-phenyl]-dimethylamine
[5-Fluoro-2-(4-fluoro-3-methoxyphenoxy)-benzyl]-dimethylamine;
[2-(4-Chlorophenoxy)-5-isopropylbenzyl]-methylamine;
{1-[2-(4-Chlorophenoxy)-5-trifluoromethylphenyl]-ethyl}-methylamine;
[2-(4-Chlorophenoxy)-4,5-dimethylbenzyl]-methylamine;
{1-[5-Chloro-2(3,4-dichlorophenoxy)phenyl]-propyl}-methylamine;
[2-(3,4-Dichlorophenoxy)-5-methylsulfanyl-benzyl]-methylamine;
{1-[2-(3,4-Dichlorophenoxy)-5-methylsulfanyl-phenyl]-ethyl}-methylamine;
{1-[2-(3,4-Dichloro-phenoxy)-5-methylsulfanyl-phenyl]-1-methylethyl}-methylamine;
[2-(3,4-Dichlorophenoxy)-5-methylsulfanyl-benzyl]-dimethylamine;
[2-(3,4-Dichlorophenoxy)-5-methanesulfinyl-benzyl]-dimethylamine;
[2-(3,4-Dichlorophenoxy)-5-methanesulfinyl-benzyl]-methylamine;
[2-(3,4-Dichlorophenoxy)-5-methanesulfonyl-benzyl]-methylamine;
[2-(3,4-Dichlorophenoxy)-5-methanesulfonyl-benzyl]-dimethylamine;
[2-(3,4-Dichlorophenoxy)-5-(propane-2-sulfonyl)-benzyl]-methylamine;
2-[2-(3,4-Dichlorophenoxy)-5-fluorophenyl]-piperidine;
2-[2-(3,4-Dichlorophenoxy)-5-fluorophenyl]-1-methyl-piperidine;
3-[2-(3,4-Dichlor-phenoxy)-5-fluorophenyl]-4-methyl-morpholine;
2-[2-(3,4-Dichlorophenoxy)-5-fluorophenyl]-1,2-dimethyl-piperidine;
{1-[2-(3,4-Dichlorophenoxy)-5-fluorophenyl]-cyclopropyl}-dimethylamine;
2-[2-(3,4-Dichlorophenoxy)-5-fluorophenyl]-1,5-dimethyl-pyrrolidine;
3-[2-(3,4-Dichlorophenoxy)-5-fluorophenyl]-4-methyl-thiomorpholine;
{1-[2-(3,4-Dichlorophenoxy)-5-fluorophenyl]-cyclopentyl}-methylamine;
{1-[2-(3,4-Dichlorophenoxy)-5-(propane-2-sulfonyl)-phenyl]-ethyl}-methylamine; and
[4-Chloro-2-(3,4-dichlorophenoxy)-5-methanesulfonyl-benzyl]-methylamine.

In other embodiments, this invention relates to the above pharmaceutical compositions for the treatment of depression or anxiety, and the above methods of treating depression or anxiety, wherein the compound of formula XXX, or pharmaceutically acceptable salt thereof, is a compound wherein m is zero, n is one, R³ and R⁴ are hydrogen, X is chloro, bromo, iodo or methyl, R¹ is hydrogen and R² is methyl.

An example of a dopamine D3 receptor agonist that may be used in the methods and pharmaceutical compositions of this invention is pramipexole ((6S)-4,5,6,7-tetrahydro-N6-propyl-2,6-benzothiazolediamine, Mirapex®) (also pramipexole dihydrochloride, SND 919, U 98528E, SND 919CL2Y, PNU 98528E, Sifrol, Daquiran and Mirapexin), as depicted below, and its pharmaceutically acceptable salts. 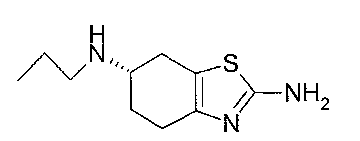 The activity and synthesis of pramipexole is referred to in United States Patent Nos. 4,843,086 and 4,886,812, issued June 27, 1989 and December 12, 1989, respectively, (product patents), all of which are incorporated herein by reference in their entirety.

Another example of a dopamine D3 receptor agonist that may be used in the methods and pharmaceutical compositions of this invention is ropinirole (4-[2-(dipropylamino)ethyl]-1,3-dihydro-2H-indol-2-one, ReQuip®) (also ropinirole hydrochloride, ropinerole, SKF 101468 and SKF 101468A), as depicted below, and its pharmaceutically acceptable salts. 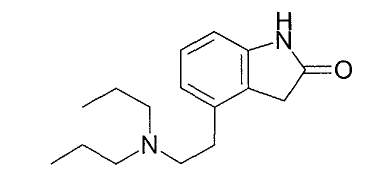 The activity and synthesis of ropinirole is referred to in United States Patent Nos. 4,452,808 and 4,588,740, issued June 5, 1984 and May 13, 1986, respectively (product patents), in United States Patent Nos. 4,950,765, issued August 21, 1990, 4,997,954, issued March 5, 1991, and 5,336,781, issued August 9, 1994 (process patents), and in International Patent Application No. WO 91/16306, published October 31, 1991 (process patent), all of which are incorporated herein by reference in their entirety. The activity of ropinirole is also referred to in Bowen WP, Coldwell MC, Hicks FR, Riley GJ, Fears R, "Ropinirole, a novel dopaminergic agent for the treatment of Parkinson' disease, with selectivity for cloned dopamine D3 receptors," British Journal Of Pharmacology, 110 Proc Suppl 93 (1993), and in Coldwell MC, Hagan J, Middlemiss D, Tulloch I, Boyfield I, "Ropinrole is a DE selective agonist at cloned human dopamine D2 long, D3 and D4.4 receptors in functional studies using microphysiometry," British Journal Of Pharmacology, 119 Supp 346 (1996).

Another example of a dopamine D3 receptor agonist that may be used in the methods and pharmaceutical compositions of this invention is cabergoline ((8β)-N-[3-(dimethylamino)propyl]-N-[(ethyl-amino)carbonyl]-6-(2-propenyl)-ergoline-8-carboxamide, Dostinex®) (also FCE 21336, CG 101, Sogilen, Cabaser®), as depicted below, and its pharmaceutically acceptable salts. 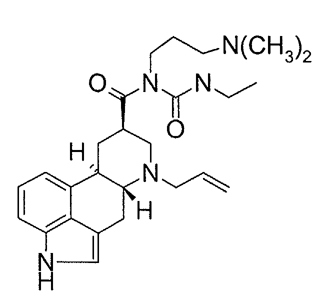 The activity and synthesis of cabergoline is referred to in United States Patent No. 4,526,892, issued July 2, 1985 (product patent), which is incorporated herein by reference in its entirety.

Another example of a dopamine D3 receptor agonist that may be used in the methods and pharmaceutical compositions of this invention is L-etrabamine (4,5,6,7-tetrahydro-N6-methyl-6-benzothiazolamine), as depicted below, and its pharmaceutically acceptable salts. 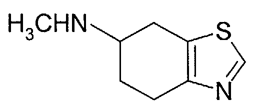 The activity and synthesis of L-etrabamine is referred to in United States Patent No. 4,208,420, issued June 17, 1980, which is incorporated herein by reference in its entirety.

Another example of a dopamine D3 receptor agonist that may be used in the methods and pharmaceutical compositions of this invention is 7-hydroxy-DPAT (R(+)-7-hydroxy-2-dipropylaminotetralin), as depicted below, and its pharmaceutically acceptable salts.

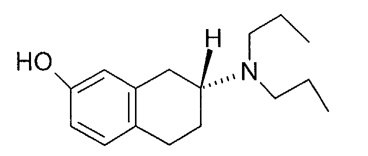

Another example of a dopamine D3 receptor agonist that may be used in the methods and pharmaceutical compositions of this invention is PD-128,907 (*trans*-(+/-)-3,4,4a,10b-tetrahydro-4-propyl-2H,5H-[1]-benzopyrano[4,3-b]-1,4-oxazin-9-ol), as depicted below, and its pharmaceutically acceptable salts. 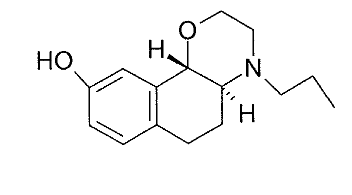 The activity and synthesis of PD-128,907 is referred to in United States Patent No 4,420,480, issued December 13, 1983, which is incorporated herein by reference in its entirety.

Another example of a dopamine D3 receptor agonist that may be used in the methods and pharmaceutical compositions of this invention is S-33592 ((3aα,9bβ)-2(4-acetylaminobenzyl)-8-cyano-1,2,3,4,4a,9b-hexahydrochromeno[3,4-c]pyrrole), as depicted below, and its pharmaceutically acceptable salts. 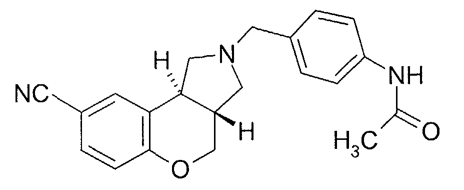 The activity and synthesis of S-33592 is referred to in United States Patent No. 6,090,837, issued July 18, 2000, which is incorporated herein by reference in its entirety.

The term "treating", as used herein, refers to reversing, alleviating, or inhibiting the progress of the disease, disorder or condition, or one or more symptoms of such disease, disorder or condition, to which such term applies. Depending on the condition of the patient, as used herein,- this term also refers to preventing a disease, disorder or condition, and includes preventing the onset of a disease, disorder or condition, or preventing the symptoms associated with a disease, disorder or condition. As used herein, this term also refers reducing the severity of a disease, disorder or condition or symptoms associated with such disease, disorder or condition prior to affliction with the disease, disorder or condition. Such prevention or reduction of the severity of a disease, disorder or condition prior to affliction refers to administration of the composition of the present invention, as described herein, to a subject that is not at the time of administration afflicted with the disease, disorder or condition. "Preventing" also refers to preventing the recurrence of a disease, disorder or condition or of symptoms associated with a disease, disorder or condition. The terms "treatment" and "therapeutically" refer to the act of treating, as defined above.

The term "mammal", as used herein, refers to any member of the class "Mammalia", including, but not limited to, humans, dogs and cats.

The pharmaceutical compositions and methods of this invention comprise, or comprise administering, monoamine reuptake inhibitors of formulas I and XXX through XXXII, which may have chiral centers and therefore exist in different enantiomeric forms. This invention includes methods and pharmaceutical compositions, as described above, wherein the monoamine reuptake inhibitors (*i*.*e*., the compounds of formulas I, XXX, XXXI and XXXII) that are employed are optical isomers, tautomers or stereoisomers of the compounds of formulas I and XXX through XXXII, as defined above, or mixtures thereof.

Those monoamine reuptake inhibitors of formulas I and XXX through XXXII that contain basic groups can form acid addition salts with various inorganic and organic acids. The present invention also relates to pharmaceutical compositions and methods comprising, or comprising administering, pharmaceutically acceptable acid addition salts of monoamine reuptake inhibitors of formulas I and XXX through XXXII and of dopamine D3 receptor agonists. The chemical acids that may be used as reagents to prepare the pharmaceutically acceptable acid salts of the basic active agents that are employed in the methods and pharmaceutical compositions of this invention are those that form non-toxic acid addition salts with such compounds. Such non-toxic salts include, but are not limited to those derived from salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [*i*.*e*., 1,1'-methylene-bis-(2-hydroxy-3- naphthoate)]salts.

Those monoamine reuptake inhibitors of formulas I and XXX through XXXII that contain acidic groups can form base addition salts with certain bases. The present invention also relates to pharmaceutical compositions and methods comprising, or comprising administering, pharmaceutically acceptable base addition salts of monoamine reuptake inhibitors of formulas I and XXX through XXXII and of dopamine D3 receptor agonists. The chemical bases that may be used as reagents to prepare the pharmaceutically acceptable base salts of the acidic active agents that are employed in the methods and pharmaceutical compositions of this invention are those that form non-toxic base salts with such compounds. Such non-toxic base salts include, but are not limited to, those derived from such pharmacologically acceptable cations such as alkali metal cations *(e.g.*, potassium and sodium) and alkaline earth metal cations (*e.g.*, calcium and magnesium), ammonium or water-soluble amine addition salts such as N-methylglucamine (meglumine), and the lower alkanolammonium and other base salts of pharmaceutically acceptable organic amines.

The present invention also relates to pharmaceutical compositions and methods of treatment that employ isotopically-labeled compounds that are identical to those recited in formulas I and XXX through XXXII but for the fact that one or more atoms are replaced by an atom having an atomic mass or mass number different from the atomic mass or mass number usually found in nature. Examples of isotopes that can be incorporated into the monoamine reuptake inhibitors of formulas I and XXX through XXXII that are employed in the pharmaceutical compositions and methods of the present invention include isotopes of hydrogen, carbon, nitrogen, oxygen, phosphorous, sulfur, fluorine and chlorine, such as ²H, ³H, ¹³C, ¹¹C, ¹⁴C, ¹⁵N, ¹⁸O, ¹⁷O, ³¹P, ³²P, ³⁵S, ¹⁸F, and ³⁶Cl, respectively. The monoamine reuptake inhibitors of formulas I and XXX through XXXII employed in the pharmaceutical compositions and methods of the present invention, prodrugs thereof, and pharmaceutically acceptable salts of such compounds or of such prodrugs which contain the aforementioned isotopes and/or other isotopes are within the scope of this invention. Such compounds may be useful as research and diagnostic tools in metabolism pharmacokinetic studies and in binding assays. Certain isotopically-labeled monoamine reuptake inhibitors of formulas I and XXX through XXXII, for example, those into which radioactive isotopes such as ³H and ¹⁴C are incorporated, are useful in drug and/or substrate tissue distribution assays. Tritiated, *i*.*e*., ³H, and carbon-14, *i.e.,* ¹⁴C, isotopes are particularly preferred for their ease of preparation and detectability. Further, substitution with heavier isotopes such as deuterium, *i.e.,* ²H, can afford certain therapeutic advantages resulting from greater metabolic stability, for example increased *in vivo* half-life or reduced dosage requirements and, hence, may be preferred in some circumstances. Isotopically-labeled monoamine reuptake inhibitors of formulas I and XXX through XXXII and prodrugs thereof can generally be prepared by carrying out the procedures disclosed in the schemes and discussion of the schemes below and substituting a readily available isotopically-labeled reagent for a nonisotopically-labeled reagent.

### Detailed Description Of The Invention

In the discussion that follows, formulas I and XXX through XXXII are defined as set forth above in the Summary of the Invention.

Compounds of the formula I and their pharmaceutically acceptable salts can be prepared as described below and in U.S. Patent Application No. 09/692,335, filed October 19, 2000, entitled "Novel Biaryl Ether Derivatives Useful As Monoamine Reuptake Inhibitors," and International Patent Application No. PCT/IB00/01373, filed September 25, 2000, each claiming priority from U.S. Provisional Application No. 60/167,761, filed November 29, 1999. The foregoing patent applications are incorporated herein by reference in their entirety.

Schemes 1-5 below and the discussion of Schemes 1-5 that follows illustrate methods of preparing compounds of the formula I. Unless otherwise indicated, in Schemes **1-5** and the discussion of Schemes **1-5** that follows, A, B, R¹, R², R³, R⁴, R⁵, R⁶, X, Y, Z, m, n and p are as defined above for compounds of the formula I.

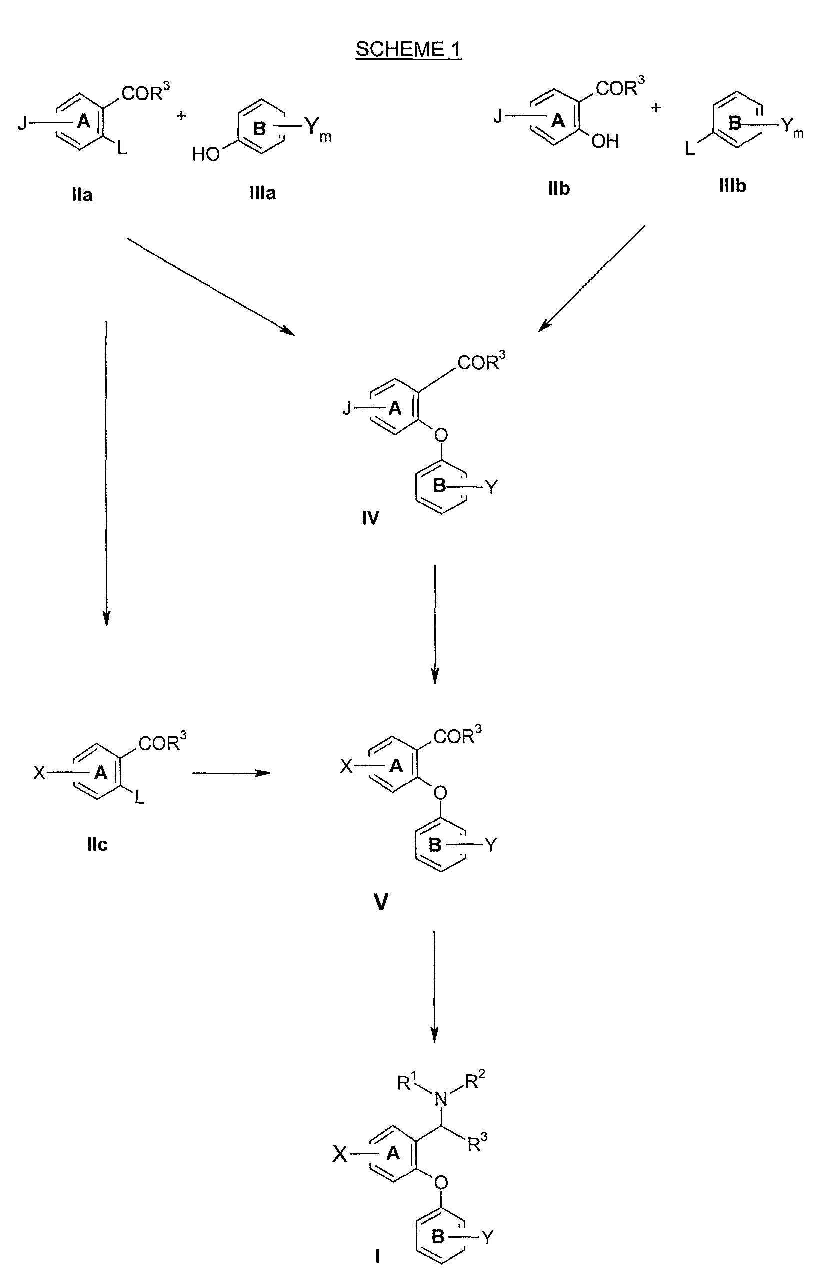

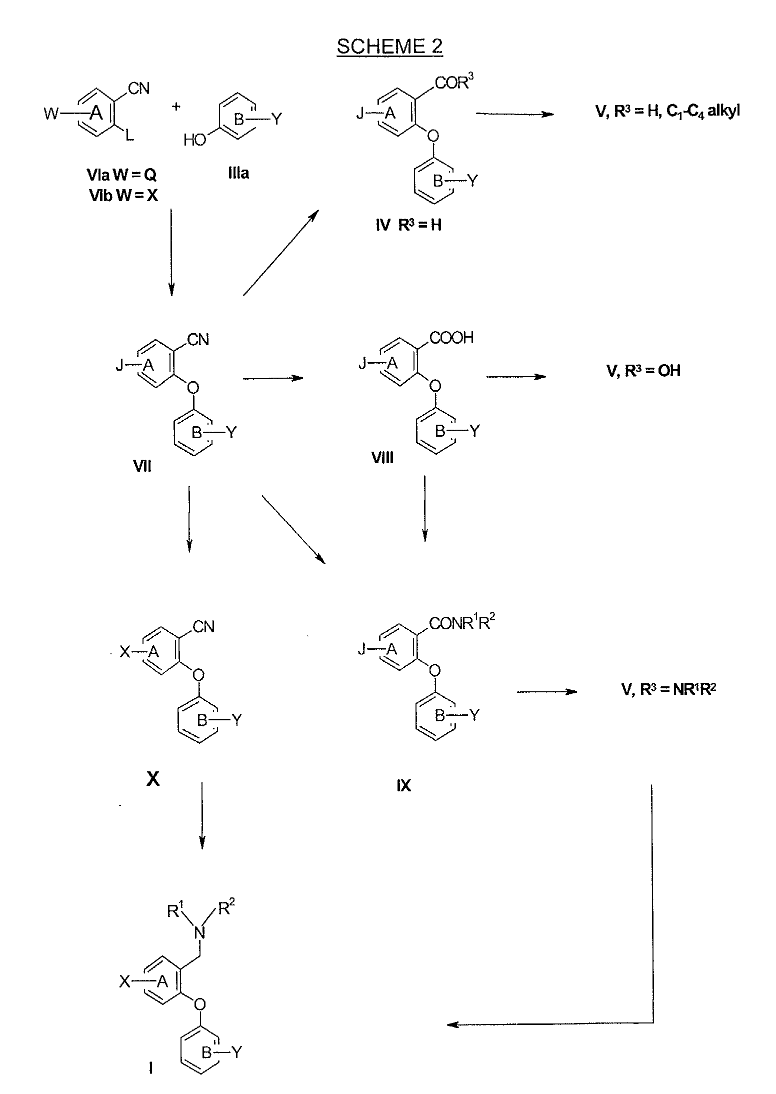

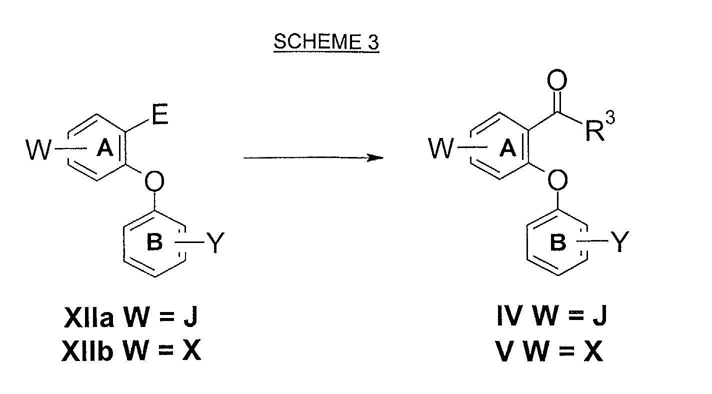

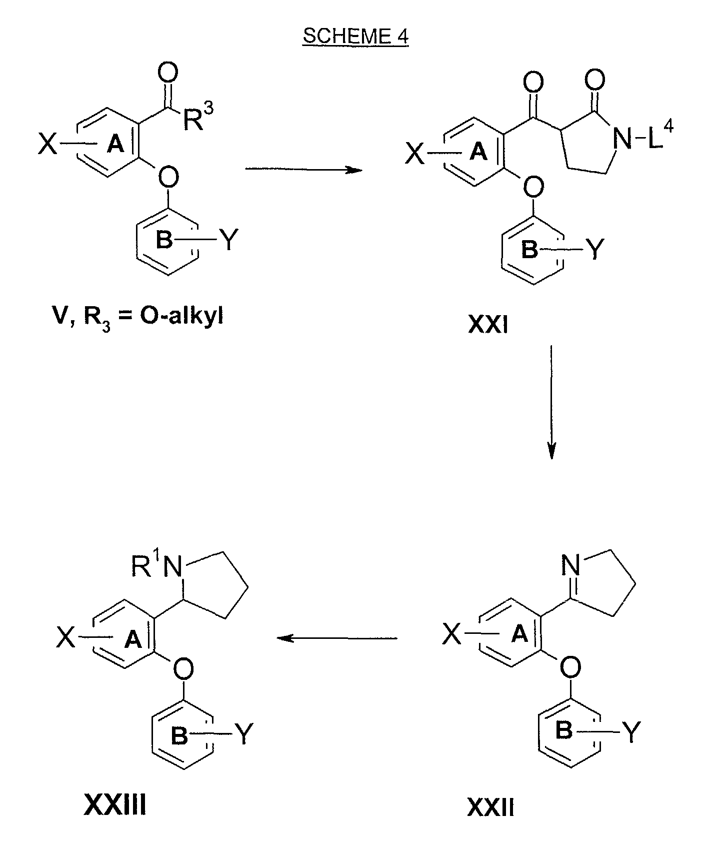

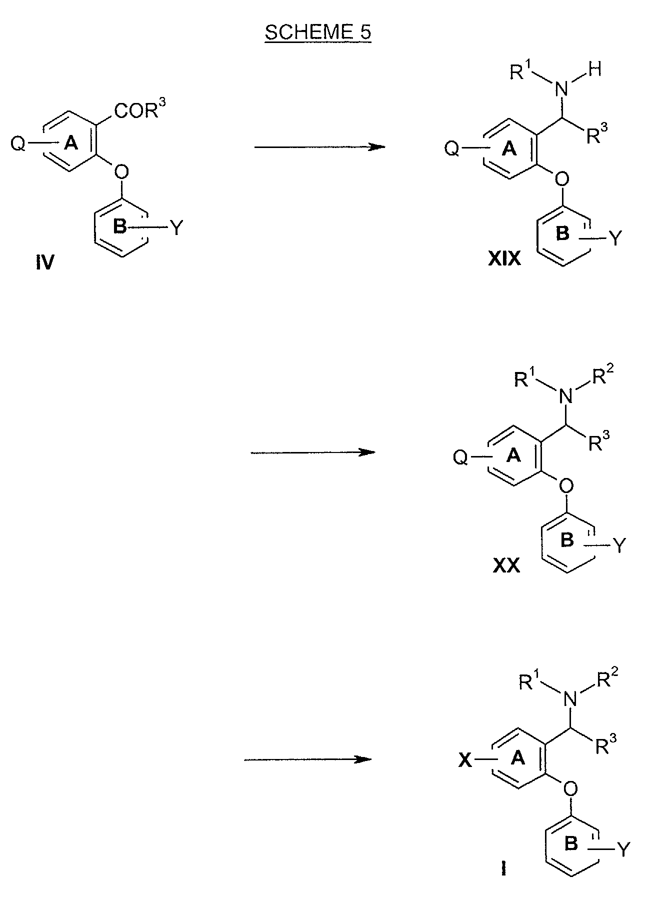

Scheme 1 refers to the preparation of compounds of the formula **I** from compounds of the formulae **II** and **III.** L represents a suitable leaving group such as fluoro, chloro, nitro, or triflate. In scheme 1, Z is hydrogen. However, using the appropriate starting compound of formula **II,** compounds of formula **I** wherein Z is other than hydrogen can be prepared according to the same scheme. Compounds of the formulas **IIa, IIb, IIIa** and **IIIb** are commercially available or can be made by methods well known to those of ordinary skill in the art. For example, compounds of general formulas **IIa** and **IIb** wherein R³ is H may be prepared by introducing an aldehyde functional group (CHO) to a compound of formula **XV** or **XVI,** respectively, using methods well known to those of skill in the art.

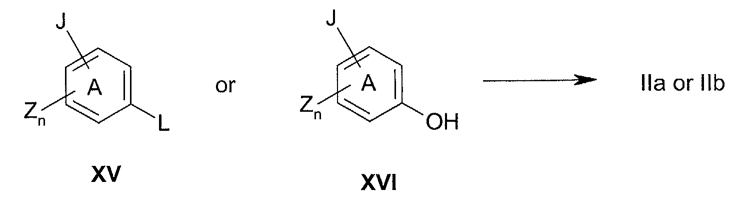

When L = F, the procedure of A. J. Bridges et al., Tetrahedron Letters, **1992,** 33(49), 7499-7502, is particularly useful for the synthesis of substituted ortho-fluorobenzaldehydes. Other such transformations have been described by C. F. H. Allen et al., Organic Synthesis, **1951**, 31, 92; T. DePaulis et al, Journal of Medicinal Chemistry, **1986,** 29, 61; I. M. Godfrey et al., J. Chemical Society, Perkin Transactions 1, **1974,** 1353; K. M. Tramposil et al., Journal of Medicinal Chemistry, **1983,** 26(2), 121; and M. E. Cracknell et al., Chemistry and Industry, (London), **1985,** (9), 309.

Referring to Scheme 1, a compound (i.e., an aldehyde or ketone) of the formula **IIa** is reacted with a compound (i.e., a phenol) of the formula **IIIa** in the presence of a base to form the corresponding compound of formula **IV.** This reaction is generally carried out at a temperature from about 0°C to about 150°C for about 1 hour to about 3 days, preferably at about 90-95°C for about 18 hours, in a polar solvent such as dimethyl sulfoxide (DMSO), N,N-dimethylformamide (DMF), N,N-dimethylacetamide (DMA) or N-methyl-2-pyrrolidinone (NMP), preferably DMF. Suitable bases include anhydrous sodium carbonate (Na₂CO₃), potassium carbonate (K₂CO₃), sodium hydroxide (NaOH), potassium hydroxide (KOH) and amines such as pyrrolidine, triethylamine and pyridine, with anhydrous K₂CO₃ being preferred. Details for conducting this procedure can be found in G. W. Yeager et al., Synthesis, **1995,** 28-30; J. R. Dimmock et al., Journal of Medicinal Chemistry, **1996,** 39(20), 3984-3997. Alternatively, phenols of the formula **IIb** and compounds of the formula **IIIb** may be converted into aldehydes or ketones of the general formulae **IV** according to the procedures described by K. Tomisawa et al., Chemical and Pharmaceutical Bulletin, **1984,** 32(8), 3066-3074.

Next, a compound of the formula **IV,** wherein J is a leaving group, for example bromine, iodine, triflate, fluorosulfonate or methanesulfonate, can be converted to a compound of the formula **V** by reaction with a compound of the general formula **X-G,** wherein G is defined as a reactive leaving group such as B(OH)₂, Sn[(C₁-C₆)alkyl], Zn(Hal) and the like, usually in the presence of a catalytic amount of a catalyst, e.g., tetrakis(triphenylphosphine) palladium(0), tetrakis(triphenylphosphine) nickel(0) or dichlorobis(triphenylphosphine) palladium(II), among others, and in the presence of a base such as sodium carbonate, potassium carbonate or triethylamine. The reactions can be conducted in a variety of organic solvents (e.g., benzene, dimethoxyethane) or in mixtures such as aqueous N,N-dimethylformamide or aqueous ethanol at temperatures in the range of about 0 °C to about 100 °C. A good general reference for this process may be found in the review by Stephen Stanforth, Tetrahedron, **1998,** 54, 263-303. Other specific references include M. J. Sharp et al, Synthetic Communications, **1981,** 11(7), 513; R. B. Miller et al, Tetrahedron Letters, **1989,** 30(3), 297; W. J. Thompson et al, Journal of Organic Chemistry, **1984,** 49(26), 5237. The compounds of the general formula **X-G** are in many cases commercially available or can be prepared by one skilled in the art of organic synthesis (for example, see the procedures in M.J. Sharp and V. Snieckus, Tetrahedron Letters, **1985,** 26(49), 5997-6000; G. W. Kabalka et al, Journal of Organometallic Chemistry, **1983,** 259, 269-274).

Alternatively, an intermediate of the formula **IIa** may be converted into a compound of the formula **IIc,** wherein X is as defined above, using the methods described above for the conversion of compounds of formula **IV** to **V.** These intermediates of formula **IIc** can then be reacted with a compound of the general formula **IIIa** to produce the ethers of general formula **V** using the methods described above for the conversion of compounds of formula **IIa** to **IV.**

Additionally, compounds of formulae **IIa** or **IV,** wherein J is a functional group like CN, can be converted to compounds of the formula **IIc** or **V** wherein X is a moiety such as 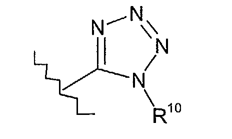 and wherein R¹⁰ is independently chosen from hydrogen, (C₁-C₆)alkyl, aryl-(C₁-C₆)alkyl or aryl, optionally substituted with hydrogen, halo, (C₁-C₆)alkyl, (C₁-C₆)alkoxyl or (C₁-C₆)SOᵣ, where r is zero, one or two. Methods for this conversion are well documented in the chemical literature; for example, through the use of sodium azide and lithium chloride in 2-methoxyethanol according to the procedure described by J. Sauer et al, Tetrahedron, **1960,** 11, 241. Under these conditions, it may be necessary to initially protect the COR³ group of compound **IIa** or **IV** to effectively convert the J group to the corresponding group X of compounds **IIc** or **V,** respectively. There are many protecting groups available which can be utilized in this process, including acetals and ketals which are described and referenced by P. G. M. Wuts and T. W. Green in Protective Groups in Organic Synthesis, 2^{nd} ed., John Wiley and Sons, New York, **1991,** pp 175-223. The selection of an appropriate protecting group can be made based upon the presence of other reactive groups in the molecule.

Subsequently, compounds of the formula **V** (R³ = H or (C₁-C₄)alkyl) can be converted into compounds of the formula **I** by subjecting them to reductive amination conditions. For example, a compound of the formula **V** can be reacted with a compound of the formula HNR¹R² to form an intermediate of the formula **XVII:** 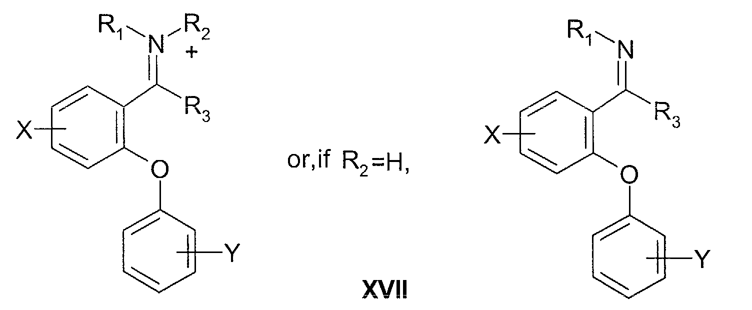 which may be isolated or converted directly in the same reaction step into a compound of the formula **I.** This conversion, whether in situ or starting with the isolated compound of formula **XVII,** can be performed using one or more methods known to those skilled in the art.

For example, the compound of formula **V** and the appropriate compound of formula HNR¹R² can be combined in the presence of a dehydrating reagent such as titanium (IV) tetrachloride or titanium (IV) isopropoxide, in a reaction inert solvent such as benzene, toluene, ethanol or a like solvent, until the reaction is judged to be complete, according to the procedure of S. Bhattarcharyya (Journal of Organic Chemistry, **1995,** 60(15), 4928-4929). Alternatively, the compound of formula **V** and the compound of formula HNR¹R² can be combined in an inert solvent such as benzene or toluene, in the presence or absence of a water scavenger such as molecular sieves, and heated to eliminate water generated during the formation of the intermediate of formula **XVII.** The degree of completion of the conversion of compounds of the formula **IV** into the above intermediate(s) of formula **XVII** can be assessed using one or more known analytical techniques, including ¹H-NMR spectroscopy

In some instances, it may be possible or desirable to isolate the intermediate(s) of formula **XVII,** or they may be further reacted with a reducing agent selective for the reduction of the intermediate to the desired compounds of formula **I**. Such reducing agents are widely known to those skilled in the art and include, for example, sodium borohydride (NaBH₄), sodium cyanoborohydride (NaBH₃CN) and sodium triacetoxy-borohydride (NaBH(OAc)₃), as described by A. F. Abdel-Magid et al. in Tetrahedron Letters, **1990,** 31, 5595. This reduction is generally carried out in a polar solvent such as methanol, ethanol, isopropanol or a like solvent, and at temperatures of about 0°C to about 100°C, preferably at room temperature. In the procedure described by Bhattarcharyya, the intermediate of formula **XVII** is formed in an ethanol solvent and, without isolation, is reduced to the product of formula **I** using NaBH₄.

As an alternative to the aldehyde or ketone intermediates of formulae **IV** and **V,** one skilled in the art can also prepare compounds of formula **VII** (i.e., nitriles), beginning with compounds of the formulae **IIIa** and **VI,** as illustrated in Scheme 2, using the diphenyl ether formation procedure described in Scheme 1. These compounds can then serve as intermediates for the syntheses of the desired compounds of formula **I.** Procedures for preparation of the compounds of formula **VI** used in this process can be adapted from those found in the literature. (See, e.g., D. C. Remy et al., Journal of Medicinal Chemistry, **1975,** 18(2), 142-148; E. A. Schmittling et al., Journal of Organic Chemistry, **1993,** 58(12), 3229-3230).

The conversion of the nitriles of formula **VII** so obtained into the desired products of formula **I** can be achieved by several routes, as depicted in Scheme 2. For example, the nitrile group of **VII** can be hydrolyzed under acidic conditions using methods well known to those of skill in the art, to produce a carboxylic acid derivative of formula **VIII.** (See, e.g., A. I. Meyers et al., Tetrahedron Letters, **1984,** 25 (28), 2941; and R. W. Higgins et al., Journal of Organic Chemistry, **1951,** 16, 1275). This carboxylic acid derivative can then be converted to a compound of the formula **V** (R³ = OH), using procedures previously described in Scheme 1 for the conversion of **IV** to **V;** subsequently compound **V** (R³ = OH) can be converted to compound **V** (R³ = NR¹R²) and then to the compounds of formula **I** as described below.

Alternatively, compound **VIII** can be converted into a carboxamide derivative of formula **IX** using one or more standard methods which are disclosed in the chemical literature, e.g., via reaction of an acid halide prepared from a compound of the formula **VIII** with an amine of general formula HNR¹R² (see R. E. Kent et al., Organic Synthesis, Coll. Vol. III, **1955,** 490; and R. M. Herbst et al., Organic Synthesis, Coll, Vol. II, **1943,** 11 for discussions of the Schotten-Bauman reaction). These carboxamides of formula **IX** can then be converted to the corresponding carboxamides of formula **V** (R³ = NR¹R²) by replacing the J substituent with the appropriate X group using conditions similar to those described for converting **IV** to **V** in Scheme 1.

The carboxamides of formulae **V** so prepared can then be reduced to the final products of formulae I using an appropriate reduction process. Depending on the presence of substituents X, Y and Z on the carboxamides **V,** this reduction can be accomplished using one or more of a variety of reagents including lithium aluminum chloride (e.g., J. Lehmann et al, Archiv. Pharm. (Weinheim, Ger.), **1982,** 315 (11), 967; N. S. Narasimhan and P. A. Patil, Journal of the Chemical Society, Chemical Communications, **1987,** (3), 191), diborane (H. C. Brown et al, Journal of the American Chemical Society, **1970,** 92, 1637 and **1973,** 38, 912; N. M. Moon et al, Journal of Organic Chemistry, **1973,** 38, 2786; H. C. Brown and V. Verma, Journal of Organic Chemistry, **1974,** 39, 1631), thexylborane / diethylaniline (A. Pelter et al, Tetrahedron Letters, **1978,** 4715), phosphorus trichloride / toluene followed by ethanolic sodium borohydride (A. Rahman et al, Tetrahedron Letters, **1976,** 219) or aluminum hydride (H. C. Brown et al, Journal of the American Chemical Society, **1966,** 88,1464; A. F. Burchat et al, Journal of Organic Chemistry, 1996, 61(21), 7627-7630).

The resulting carboxamides of the formula **IX,** wherein R¹ and R² are hydrogen, can also be prepared directly from the corresponding nitriles of formula **VII** by specific hydrolysis methods, employing, for example, hydrogen peroxide or strong aqueous alkali metal salts. (See Chemistry & Industry, **1961,** 1987; C. R. Noller, Organic Synthesis, Coll. Vol. II, **1943**, 586; and J. H. Hall and M. Gisler, Journal of Organic Chemistry, **1976,** 41, 3769). Subsequently, the carboxamide derivatives of formula **IX** may can be converted to the carboxamide compounds of formula **V** (R³ = NR¹R²) in the manner just described for the conversion of **VIII** to **V.**

Finally, the nitriles of formula **X,** obtained from the nitriles of formula **VII** analogously to the conversion of compounds of formulae **IV** to **V,** can be reduced to the desired compounds of general formula **I,** wherein R¹ and R² are both hydrogen, by using one of a variety of reducing agents disclosed in the chemical literature which are selective for this transformation (including catalytic hydrogenation using hydrogen gas and platinum (II) oxide, as described by J. A. Secrist, III and M. W. Logue in Journal of Organic Chemistry, **1972,** 37, 335; hydrazine hydrate and Raney nickel in ethanol, as described by W. W. Zajac, Jr. et al. in Journal of Organic Chemistry, **1971,** 36, 3539; and sodium trifluoroacetoxy borohydride in THF, as described by N. Umino et al. in Tetrahedron Letters, **1976,** 2875). Such reducing agents can also include lithium aluminum hydride in a nonreactive solvent such as diethyl ether or tetrahydrofuran (see, e.g., A. C. Cope et al., Organic Synthesis, Coll. Vol. IV, **1963,** 339, for use of lithium aluminum hydride in a diethyl ether or THF solvent).

The nitriles of formula **VII** may also be converted to the corresponding aldehydes of general formula **IV,** wherein R³ is hydrogen, using reagents and conditions which are specific for this transformation, such as lithium triethoxyaluminum hydride in a solvent such as THF or diethyl ether, as described by H. C. Brown and C. P. Garg in Journal of the American Chemical Society, **1964,** 86, 1085 and by J. Malek and M. Cerny in Synthesis, **1972,** 217.

In addition to the methods described above in Schemes 1 and 2 for the preparation of the intermediate aldehydes and ketones of formula **I**, other methods exist which can provide compounds of the formula **I.** For example, in the procedure depicted in Scheme 3, a compound of formula **XIIa,b,** in which E is a hydrogen atom, can be reacted, under conditions of Friedel-Crafts acylation (e.g., AlCl₃/CH₂Cl₂/R³COCl), to produce ketones of the formula **IV** or **V** in which R³ is C₁-C₄ alkyl (C. F. H. Allen, Organic Synthesis, Coll. Vol. II, 3, **1943).** Alternatively, an acid anhydride, i.e., (R³CO)₂O can be reacted under similar conditions (O. Grummitt et al, Organic Synthesis, Coll. Vol. III, 109, **1955)** to produce the intermediate compounds of formula **IV** or **V.** When it is desired to prepare compounds of formula **IV** or **V** where R³ is hydrogen, said compound may be prepared from compounds of formula **XIIa,b** via a Vilsmeier-Haack acylation, using the methods described by E. Campaigne and W. L. Archer, Organic Synthesis, Coll. Vol. IV, 331, **1963** and by J. H. Wood and R. W. Bost, Organic Synthesis, Coll. Vol. IV, 98, **1955.**

The location of the acyl group (COR³) introduced in this manner can be determined by the nature and location of the J, X and/or Y substituents present, as well as by the conditions employed for the reaction. In instances where it is desirable to prepare compounds of formula **IV** (R³ =H), from **Xlla** (E=H), introduction of the aldehyde functional group (CHO) can also be achieved using conditions described above for the preparation of the intermediates **IIa** and **IIb** in Scheme 1. For example, preparation of compounds of the formula **IV** wherein R³ = H (i.e., aldehydes) can be achieved using one or more of the known procedures for the formylation of aromatic rings, including reacting dichloromethyl methyl ether and titanium (IV) tetrachloride in methylene chloride according to the procedure described by M. L. Mancini et al., Synthetic Communications, **1989,** 2001-2007, or H. Chikashita et al., Journal of Organic Chemistry, **1991**, 56, 1692.

For the preparation of compounds of the general formula **I** wherein R² and R³ taken together with the nitrogen to which R² is attached and the carbon to which R³ is attached form a nitrogen containing ring, an adaptation of the procedure described by L. S. Bleicher et al (Journal of Organic Chemistry, **1998,** 63, 1109) can be employed, as shown in Scheme 4. Thus, an ester of the general formula **V** (R³ = O-alkyl), prepared by esterification of the corresponding carboxylic acid of formula **V** (R³ = OH), is reacted with a cyclic lactam of the general formula **XXV** 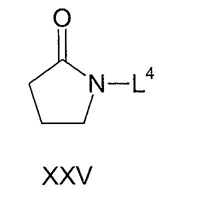 where L⁴ is a reaction labile group such as -CH=CH₂, in the presence of a strong base such as sodium methoxide, to produce the diketo intermediate of general formula **XXI.** This intermediate can then be converted to the corresponding cyclic imine of formula **XXII** in the presence of a strong acid, such as hydrochloric acid, usually under reflux conditions. Subsequently, the compounds of formula **XXII** can be reduced to form the cyclic amines of formula **XXIII** (wherein R¹ = H) using, for example, sodium borohydride in methanol as described previously. Such compounds of formula **XXIII** can further be converted into compounds of the formula **XXIII** (wherein R¹ is as defined for compounds of formula **I**) as previously discussed.

For the preparation of compounds of general formula **I,** wherein the group X is a lactam attached to phenyl or naphthyl ring A via the lactam N atom, the method illustrated in Scheme 5 is preferred. In this procedure an aldehyde or ketone of general formula **IV** (R³ = H or C₁-C₄ alkyl, respectively) where Q is NO₂ is converted to an amine of the general formula **XIX** where R¹ is as previously defined, according to the method described in Scheme 1. This intermediate **XIX** is then converted to a compound of general formula **XX,** where R² is a protecting group, preferably a *tert*-butoxy-carbonyl (*t*-BOC) group, that is stable to hydrogenation conditions but can be readily removed at a later point in the synthetic sequence; suggestions for such groups can be found in Wuts and Green, supra, at page 309. This latter intermediate **XX** wherein Q is NO₂ can then be treated under reduction conditions to form an analogous intermediate of formula **XX** wherein Q is NH₂, while leaving the *t*-BOC group intact. Such reduction conditions for this conversion are known to one skilled in the art and include the use of hydrogen gas (H₂) and a catalyst, preferably palladium on carbon, in a reaction inert solvent such as a lower alcohol (e.g., methanol, ethanol), ester (e.g., ethyl acetate), or ether (e.g., tetrahydrofuran, 1,4-dioxane) and in the presence or absence of a small amount of acid, preferably a small amount of acetic acid. The NH₂ group of the resulting compounds of formula **XX** can then be converted to cyclic amides (lactams), wherein R² remains *t*-BOC, by reacting them with an omega-chloro alkanoyl chloride or bromide or an omega-bromo alkanoyl chloride or bromide in a neutral solvent such as THF and in the presence of an acid scavenger, such as Na₂CO₃, K₂CO₃, CS₂CO₃ or the like, and heating the mixture at the boiling point of the solvent. This effects a ring closure forming the cyclic amide (i.e. lactam). Finally, the protecting group can be removed to obtain the compounds of general formula **I** wherein X is a lactam and R² is H; in the case of the *t*-BOC protecting group a mixture of ethyl acetate saturated with HCI gas is effective in such removal.

Compounds of formula XXX through XXXII and their pharmaceutically acceptable salts can be prepared as described in U.S. Patent Application No. 09/529,207, filed April 7, 2000, entitled "Monoamine Reuptake Inhibitors For Treatment of CNS Disorders," and International Patent Application No. PCT/IB00/00108, filed February 2, 2000, and published as International Patent Application No. WO 00/50380 on August 31, 2000 (of which U.S. Patent Application No. 09/529,207 is the national stage application), which claims priority from U.S. Provisional Application No. 60/121,313, filed February 23, 1999. The foregoing patent applications are incorporated herein by reference in their entirety.

This invention relates both to methods of treating depression or anxiety in which the monoamine reuptake inhibitor (*i*.*e*., the compound of formula I, XXX, XXXI or XXXII) and the dopamine D3 receptor agonist, or pharmaceutically acceptable salts of the same, are administered together, as part of the same pharmaceutical composition, as well as to methods in which these two active agents are administered separately as part of an appropriate dose regimen designed to obtain the benefits of the combination therapy. The appropriate dose regimen, the amount of each dose administered, and specific intervals between doses of each active agent will depend upon the subject being treated, the emetogen and the severity of the condition. Generally, in carrying out the methods of this invention, the monoamine reuptake inhibitor (*i*.*e*., the compound of formula I, XXX, XXXI or XXXII) will be administered to a patient in an amount ranging from about 0.01 to about 10.0 mg per kg of body weight per day. As an example, a monoamine reuptake inhibitor (*i*.*e*., a compound of formula I, XXX, XXXI or XXXII) will be administered to an adult human of average weight (about 70 kg) in an amount ranging from about 0.7 mg to about 700 mg per day, preferably from about 1 mg to about 500 mg per day, in single or divided doses. The compounds may be administered on a regimen of up to 6 times per day, preferably 1 to 4 times per day, especially 2 times per day and most especially once daily. A suitable dosage level for the dopamine D3 receptor agonist is about 0.05 mg to about 1500 mg per day, preferably about 1 mg to about 500 mg per day, in single or divided doses. The compounds may be administered on a regimen of up to 6 times per day, preferably 1 to 4 times per day, especially 2 times per day and most especially once daily. Variations may nevertheless occur depending upon the species of animal being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effect, provided that such larger doses are first divided into several small doses for administration throughout the day.

It will be appreciated that the amount of the monoamine reuptake inhibitor (*i*.*e*., the compound of formula I, XXX, XXXI or XXXII) and the dopamine D3 receptor agonist required for use in the treatment of depression or anxiety will vary not only with the particular compounds or compositions selected, but also with the route of administration, the nature of the condition being treated, and the age and condition of the patient, and will ultimately be at the discretion of the patient's physician or pharmacist.

When administered in combination, either as a single or as separate pharmaceutical composition(s), the monoamine reuptake inhibitor (*i*.*e*., the compound of formula I, XXX, XXXI or XXXII) and the dopamine D3 receptor agonist, are present in a ratio which is consistent with the manifestation of the desired effect. In particular, the ratio by weight of the monoamine reuptake inhibitor (*i*.*e*., the compound of formula I, XXX, XXXI or XXXII) and the dopamine D3 receptor agonist will suitably be between 0.001 to 1 and 1000 to 1, and especially between 0.01 to I and 100 to 1.

The monoamine reuptake inhibitors (*i*.*e*., the compounds of formula I, XXX, XXXI and XXXII), their pharmaceutically acceptable salts, and the dopamine D3 receptor agonists and their pharmaceutically acceptable salts that are employed in the pharmaceutical compositions and methods of this invention are hereinafter also referred to as "therapeutic agents". The therapeutic agents can be administered via either the oral or parenteral route. Compositions containing both a monoamine reuptake inhibitor (*i*.*e*., a compound of formula I, XXX, XXXI or XXXII) and a dopamine D3 receptor agonist, or pharmaceutically acceptable salts of one or both therapeutic agents, will generally be administered orally or parenterally daily, in single or divided doses, so that the total amount of each active agent administered falls within the above guidelines.

The therapeutic agents may be administered alone or in combination with pharmaceutically acceptable carriers or diluents by either of the routes previously indicated, and such administration may be carried out in single or multiple doses. More particularly, the therapeutic agents of this invention can be administered in a wide variety of different dosage forms, *i*.*e*., they may be combined with various pharmaceutically acceptable inert carriers in the form of tablets, capsules, lozenges, troches, hard candies, suppositories, aqueous suspensions, injectable solutions, elixirs, syrups, and the like. Such carriers include solid diluents or fillers, sterile aqueous media and various non-toxic organic solvents, *etc.* Moreover, oral pharmaceutical compositions can be suitably sweetened and/or flavored. In general, the therapeutic agents of this invention, when administered separately (*i*.*e*., not in the same pharmaceutical composition) are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a therapeutic agent in either sesame or peanut oil or in aqueous propylene glycol may be employed. The aqueous solutions should be suitably buffered if necessary and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art.

As stated above, the monoamine reuptake inhibitor (*i*.*e*., the compound of formula I, XXX, XXXI or XXXII) and the dopamine D3 receptor agonist may be formulated in a single pharmaceutical composition or alternatively in individual pharmaceutical compositions for simultaneous, separate or sequential use in accordance with the present invention.

Preferably the compositions according to the present invention, which contain both a monoamine reuptake inhibitor (*i.e.*, a compound of formula I, XXX, XXXI or XXXII) and a dopamine D3 receptor agonist, as well as the pharmaceutical compositions used to deliver only one of these active agents, are in unit dosage forms such as tablets, pills, capsules, powders, granules, solutions or suspensions, or suppositories, for oral, parenteral or rectal administration, by inhalation or insufflation or administration by transdermal patches or by buccal cavity absorption wafers.

For preparing solid compositions such as tablets, the principal active ingredient is mixed with a pharmaceutical carrier, e.g., conventional tableting ingredients such as corn starch, lactose, sucrose, sorbitol, talc, stearic acid, magnesium stearate, dicalcium phosphate or gums, and other pharmaceutical diluents, *e.g.*, water, to form a solid preformulation composition containing a homogeneous mixture of a compound of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing, typically, from about 0.05 mg to about 500 mg of each of the therapeutic agents contained in the composition. The tablets or pills of the composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer that serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac acetyl alcohol and cellulose acetate.

The liquid forms in which the novel compositions of the present invention may be incorporated for administration orally or by injection include aqueous solutions, suitably flavored syrups, aqueous or oil suspensions, and flavored emulsions with edible oils such as cottonseed oil, sesame oil, coconut oil, peanut oil or soybean oil, as well as elixirs and similar pharmaceutical vehicles. Suitable dispersing or suspending agents for aqueous suspensions include synthetic and natural gums such as tragacanth, acacia, alginate, dextran, sodium carboxymethyl cellulose, methylcellulose, polyvinyl-pyrrolidone or gelatin.

Preferred compositions for administration of a monoamine reuptake inhibitor (*i*.*e*., a compound of formula I, XXX, XXXI or XXXII), or other therapeutic agent by injection include those comprising the therapeutic agent in association with a surface-active agent (or wetting agent or surfactant) or in the form of an emulsion (as a water-in-oil or oil-in-water emulsion).

Suitable surface-active agents include, in particular, non-ionic agents, such as polyoxyethylenesorbitans (*e*.*g*., Tween™ 20, 40, 60, 80 or 85) and other sorbitans (*e*.*g*., Span™ 20, 40, 60, 80 or 85). Compositions with a surface-active agent will conveniently comprise between 0.05 and 5% surface-active agent, and preferably between 0.1 and 2.5%. It will be appreciated that other ingredients may be added, for example mannitol or other pharmaceutically acceptable vehicles, if necessary.

Suitable emulsions may be prepared using commercially available fat emulsions, such as Intralipid™, Liposyn ™, Infonutrol™, Lipofundin ™ and Lipiphysan™. The therapeutic agent may be either dissolved in a pre-mixed emulsion composition or, alternatively, it may be dissolved in an oil (*e.g.*, soybean oil, safflower oil, cottonseed oil, sesame oil, com oil or almond oil) and an emulsion formed upon mixing with a phospholipid (*e.g.*, eggs phospholipids, soybean phospholipids or soybean lecithin) and water. It will be appreciated that other ingredients may be added, for example glycerol or glucose, to adjust the tonicity of the emulsion. Suitable emulsions will typically contain up to 20% oil, for example, between 5 and 20%. The fat emulsion will preferably comprise fat droplets between 0.1 and 1.0 µm, particularly 0.1 and 0.5 µm, and have a pH in the range of 5.5 to 8.0.

Compositions for inhalation or insufflation include solutions and suspensions in pharmaceutically acceptable aqueous or organic solvents, or mixtures thereof, and powders. The liquid or solid compositions may contain suitable pharmaceutically acceptable excipients as set out above. Preferably the compositions are administered by the oral or nasal respiratory route for local or systemic effect. Compositions in preferably sterile pharmaceutically acceptable solvents may be nebulised by use of inert gases. Nebulised solutions may be breathed directly from the nebulising device or the nebulising devise may be attached to a facemask, tent or intermittent positive pressure breathing machine. Solution, suspension, or powder compositions may be administered, preferably orally or nasally, from devices that deliver the formulation in an appropriate manner.

Compositions of the present invention may also be presented for administration in the form of transdermal patches using conventional technology. The compositions may also be administered via the buccal cavity using, for example, absorption wafers.

The present invention further provides a process for the preparation of a pharmaceutical composition comprising a monoamine reuptake inhibitor (*i*.*e*., a compound of formula I, XXX, XXXI or XXXII) and a dopamine D3 receptor agonist, or pharmaceutically acceptable salts of the same, which process comprises bringing a monoamine reuptake inhibitor (*i*.*e*., a compound of formula I, XXX, XXXI or XXXII) and the dopamine D3 receptor agonist (or the pharmaceutically acceptable salts of one or both of these therapeutic agents) into association with a pharmaceutically acceptable carrier or excipient.

The compounds of formula I and XXX through XXXII, and their pharmaceutically acceptable salts, are useful as monoamine reuptake inhibitors, *i*.*e*., they possess the ability to inhibit the reuptake of serotonin, dopamine and norepinephrine at the individual monoamine reuptake sites in mammals, and therefore, they are able to function as therapeutic agents in the treatment of the aforementioned disorders and diseases in an afflicted mammal.

The ability of the compounds of formula I and XXX through XXXII to inhibit the reuptake of serotonin, dopamine and norepinephrine at the individual monoamine reuptake sites may be determined using the following procedures. The *in vitro* activity of the compounds of formula I and XXX through XXXII at the individual monoamine reuptake sites can be determined using rat synaptosomes or HEK-293 cells transfected with the human serotonin, dopamine or norepinephrine transporter, according to the following procedure adapted from those described by S. Snyder *et al.,* (Molecular Pharmacology, 1971, 7, 66-80), D.T. Wong *et al.,* (Biochemical Pharmacology, 1973, 22, 311-322), H. F. Bradford (Journal of Neurochemistry, 1969, 16, 675-684) and D. J. K. Balfour (European Journal of Pharmacology, 1973, 23, 19-26).

Synaptosomes: Male Sprague Dawley rats are decapitated and the brains rapidly removed. The cortex, hippocampi and corpus striata are dissected out and placed in ice cold sucrose buffer, 1 gram in 20 ml of buffer (the buffer is prepared using 320 mM sucrose containing 1mg/ml glucose, 0.1mM ethylenediamine tetraacetic acid (EDTA) adjusted to pH 7.4 with tris(hydroxymethyl)-aminomethane (TRIS) base). The tissues are homogenized in a glass homogenizing tube with a Teflon™ pestle at 350 rpm using a Potters homogenizer. The homogenate is centrifuged at 1000 x g for 10 min. at 4°C. The resulting supernatant is recentrifuged at 17,000 x g for 20 min. at 4°C. The final pellet is resuspended in an appropriate volume of sucrose buffer that yielded less than 10% uptake.

Cell Preparation: HEK-293 cells transfected with the human serotonin (5-HT), norepinephrine (NE) or dopamine (DA) transporter are grown in DMEM (Dulbecco's Modified Eagle Medium, Life Technologies Inc., 9800 Medical Center Dr., Gaithersburg, MD, catalog no. 11995-065)) supplemented with 10% dialyzed FBS (Fetal Bovine Serum, from Life Technologies, catalog no. 26300-053), 2 mM L-glutamine and 250 ug/ml G418 for the 5-HT and NE transporter or 2ug/ml puromycin for the DA transporter, for selection pressure. The cells are grown in Gibco triple flasks, harvested with Phosphate Buffered Saline (Life Technologies, catalog no. 14190-136) and diluted to an appropriate amount to yield less than 10% uptake.

Neurotransmitter Uptake Assay: The uptake assays are conducted in glass tubes containing 50 uL of solvent, inhibitor or 10uM sertraline, desipramine or nomifensine for the 5-HT, NE or DA assay nonspecific uptake, respectively. Each tube contains 400 uL of [3H]5-HT (5 nM final), [3H]NE (10 nM final) or [3H]DA (5 nM final) made up in modified Krebs solution containing 100 uM pargyline and glucose (1mg/ml). The tubes are placed on ice and 50 uL of synaptosomes or cells is added to each tube. The tubes are then incubated at 37° C for 7 min. (5-HT, DA) or 10 min. (NE). The incubation is terminated by filtration (GF/B filters), using a 96-well Brandel Cell Harvester, the filters are washed with modified Krebs buffer and counted using either a Wallac Model 1214 or Wallac Beta Plate Model 1205 scintillation counter.

Determination of the *in vivo* serotonin reuptake inhibition activity and potency of action for the compounds used in the methods and pharmaceutical compositions of the present invention can be made by measuring the ability of the compound to block the depletion of serotonin in the anterior cortex induced by (+/-)-para-chloroamphetamine (PCA) in the rat, according to a procedure adapted from R. W. Fuller, H. D. Snoddy and M. L. Cohen in Neuropharmacology 23: 539-544 (1984).

Generally, male, white Sprague-Dawley rats weighing 160-230 g each are assigned to either the control (vehicle) or test groups. When the test compound is administered subcutaneously (sc) at a given dose, it is co-administered with 5 mg/kg of parachloroamphetamine (PCA). Three hours post-dose, the animals are sacrificed by decapitation and the anterior cortices are removed, wrapped in parafilm and frozen in dry ice (-78 C). When dosed orally (po), the rats are fasted the night before the experiment and then treated with the test compound at a given dose 30 minutes prior to the administration of the PCA (5 mg/kg, sc). After three hours, the animals are sacrificed and the tissues removed as above.

To determine the serotonin (5-HT) levels, the frozen tissues are homogenized with Branson sonifier in 0.5 mL of mobile phase in Eppendorf centrifuge tubes. Samples are then spun down at 11000 rpm for twenty minutes in a Sorval SH-MT rotor in a Sorval RC5C centrifuge. The supernatant thus obtained is pipetted into HPLC vials and the 5-HT levels are measured on HPLC-EC.

Interpretation of the results is as follows: Each experiment has a set of vehicle treated animals and a set of PCA-only animals. The mean 5-HT value of the PCA animals is subtracted from the mean 5-HT value of the vehicle animals. This is the signal or window of the response. The mean 5-HT value of each test group is determined, the mean of the PCA group subtracted from that, and that amount divided by the window is the per cent protection from the PCA effect for that dose. To report an ID₅₀, a line is drawn mathematically through the per cent protection values and the 50 per cent level calculated.

Compounds which are dopamine D3 receptor agonists, including the examples of specific dopamine D3 receptor agonists referred to herein, may be used in the treatment of disorders and conditions that can be treated by modulating binding to the dopamine D3 receptor. Thus, compounds which are dopamine D3 receptor agonists, including the examples of specific dopamine D3 receptor agonists referred to herein, are able to function as therapeutic agents in the treatment of the aforementioned disorders and diseases in an afflicted mammal.

The in vitro activity of dopamine D3 receptor agonists at the individual dopamine D3 receptor sites can be determined by measuring the affinity of such compounds for the dopamine D3 receptor using ¹²⁵I-iodosulpiride, according to the following procedure.

Chinese hamster ovary (CHO) cells which have been transvected with the dopamine D3 receptor are removed from their storage medium, pelletized with 1 mM EDTA (pH 7.4) at 4°C, and centrifuged at 1000 RPM for 5 minutes. The supernatant is removed and the cells are homogenized with a Polytron homogenizer for 20 seconds in a solution of 20 mL TRIS (i.e., tris(hydroxymethyl)aminomethane) containing 5 mM MgSO₄ (pH 7.4). The homogenate is centrifuged at 20,000 RPM for 10 minutes at 4°C. The resulting pellet is resuspended in a final volume of buffer solution (50 mM TRIS, 120 mM NaCI, 5 mM KCI, 2 mM CaCl₂, 5 mM MgCl₂, pH 7.4) so that the concentration of tissue in each tube is approximately 5 mg/mL.

Incubation mixtures are prepared using 100 µL of vehicle or test drug solution with 100 µL of the radioligand and 200 µL of tissue or cell solution in 12 X 75 mm polypropylene culture tubes. Incubation is run at 30°C for 30 minutes and terminated by filtration through Whatman GF/B Brandell Cell Harvester Filters coated with 0.3% polyethyleneimine (PEI). The filters are then washed with ice-cold buffer solution (50 mM TRIS, 120 mM NaCI), 4 X 4 mL, on a Brandell cell harvester.

Data interpretation: data are calculated as IC₅₀ (the concentration that inhibits 50% of the specific binding) or Ki values, where the Ki = IC₅₀/1+[L]/KD and L is the ligand concentration and KD is the affinity constant for the ¹²⁵I-iodosulpiride.

## Claims

1. A combination comprising a dopamine D3 receptor agonist and a second agent, selected from the group consisting of;
(i) a compound of the formula I, as depicted and defined below, 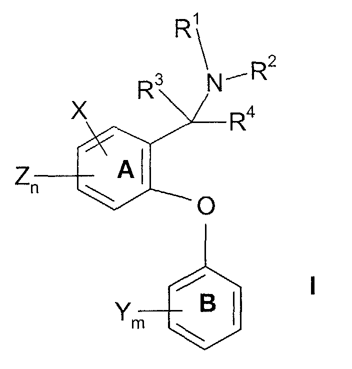 or pharmaceutically acceptable salt thereof, wherein phenyl ring A and phenyl ring B can each, independently, be replaced by a naphthyl group, and wherein when phenyl ring A is replaced by a naphthyl group, the ethereal oxygen of structure I and the carbon to which R³, R⁴ and NR¹R² are attached, are attached to adjacent ring carbon atoms of the naphthyl group and neither of said adjacent ring carbon atoms is also adjacent to a fused ring carbon atom of said naphthyl group;
n and m are selected, independently, from one, two and three;
R¹ and R² are selected, independently, from hydrogen, (C₁-C₄)alkyl, (C₂-C₄)alkenyl, and (C₂-C₄)alkynyl, or R¹ and R², together with the nitrogen to which they are attached, form a four to eight membered saturated ring containing one or two heteroatoms, including the nitrogen to which R¹ and R² are attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
R³ and R⁴ are selected, independently, from hydrogen and (C₁-C₄) alkyl optionally substituted with from one to three fluorine atoms, or R³ and R⁴ together with the carbon to which they are attached form a four to eight membered saturated carbocyclic ring, and wherein said ring may optionally be substituted at available binding sites with from one to three substituents selected, independently, from hydroxy and (C₁-C₆)alkyl;
or R² and R³, together with the nitrogen to which R² is attached and the carbon to which R³ is attached, form a four to eight membered saturated ring containing one or two heteroatoms, including the nitrogen to which R² is attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
each X is selected, independently, from phenyl, heteroaryl and heterocyclic groups, and may be further substituted by hydrogen, halo, (C₁-C₄)alkyl optionally substituted with from one to three fluorine atoms, (C₁-C₄)alkoxy optionally substituted with from one to three fluorine atoms, cyano, nitro, amino, hydroxy, carbonyl, (C₁-C₄)alkylamino, di-[(C₁-C₄)alkyl]amino, NR⁵(C=O)(C₁-C₄)alkyl, SO₂NR⁵R⁶ or SOₚ(C₁-C₆)alkyl, wherein R⁵ and R⁶ are selected, independently, from hydrogen and (C₁-C₆)alkyl, and p is zero, one or two;
each Y is selected, independently, from hydrogen, halo, (C₁-C₄)alkyl optionally substituted with from one to three fluorine atoms, (C₁-C₄)alkoxy optionally substituted with from one to three fluorine atoms, cyano, nitro, amino, (C₁-C₄)alkylamino, di-[(C₁-C₄)alkyl]amino, NR⁵(C=O)(C₁-C₄)alkyl, SO₂NR⁵R⁶ and SOₚ(C₁-C₆)alkyl, wherein R⁵ and R⁶ are selected, independently, from hydrogen and (C₁-C₆)alkyl, and p is zero, one or two; and
each Z is selected independently from hydrogen, halo, (C₁-C₄)alkyl optionally substituted with from one to three fluorine atoms, and (C₁-C₄)alkoxy;
(ii) a compound of the formula XXX, as depicted and defined below, 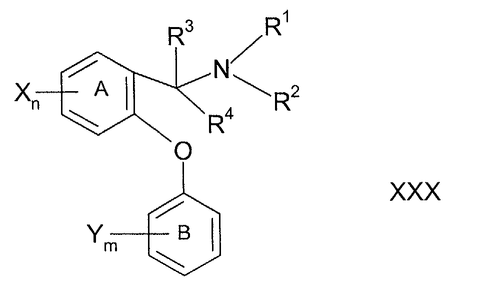 or pharmaceutically acceptable salt thereof, wherein phenyl ring A and phenyl ring B can each, independently, be replaced by a naphthyl group, and wherein when phenyl ring A is replaced by a naphthyl group, the ethereal oxygen of structure I and the carbon to which R³, R⁴ and NR¹R² are attached, are attached to adjacent ring carbon atoms of the naphthyl group and neither of said adjacent ring carbon atoms is also adjacent to a fused ring carbon atom of said naphthyl group;
n and m are, selected, independently, from one, two and three;
R¹ and R² are selected, independently, from hydrogen, (C₁-C₄)alkyl, (C₂-C₄)alkenyl, and (C₂-C₄)alkynyl, or R¹ and R², together with the nitrogen to which they are attached, form a four to eight membered saturated ring containing one or two heteroatoms, including the nitrogen to which R¹ and R² are attached, wherein the second heteroatom, when present, is selected from oxygen, nitrogen and sulfur, with the proviso that said ring can not contain two adjacent oxygen atoms or two adjacent sulfur atoms, and wherein said ring may optionally be substituted at available binding sites with from one to three substituents selected, independently, from hydroxy and (C₁-C₆)alkyl;
R³ and R⁴ are selected, independently, from hydrogen and (C₁-C₄) alkyl optionally substituted with from one to three fluorine atoms, or R³ and R⁴, together with the carbon to which they are attached, form a four to eight membered saturated carbocyclic ring, and wherein said ring may optionally be substituted at available binding sites with from one to three substituents selected, independently, from hydroxy and (C₁-C₆)alkyl;
or R² and R³, together with the nitrogen to which R² is attached and the carbon to which R³ is attached, form a four to eight membered saturated ring containing one or two heteroatoms, including the nitrogen to which R² is attached, wherein the second heteroatom, when present, is selected from oxygen, nitrogen and sulfur, with the proviso that said ring can not contain two adjacent oxygen atoms or two adjacent sulfur atoms, and wherein said ring may optionally be substituted at available binding sites with from one to three substituents selected, independently, from hydroxy and (C₁-C₆)alkyl;
each X is selected, independently, from hydrogen, halo, (C₁-C₄)alkyl optionally substituted with from one to three fluorine atoms, (C₁-C₄)alkoxy optionally substituted with from one to three fluorine atoms, cyano, nitro, amino, (C₁-C₄)alkylamino, di-[(C₁-C₄)alkyl]amino, NR⁵(C=O)(C₁-C₄)alkyl, SO₂NR⁵R⁶ and SOₚ(C₁-C₆)alkyl, wherein R⁵ and R⁶ are selected, independently, from hydrogen and (C₁-C₆)alkyl, and p is zero, one or two; and
each Y is selected, independently, from hydrogen, (C₁-C₆)alkyl and halo;
with the proviso that: (1) no more than one of NR¹R², CR³R⁴ and R²NCR³ can form a ring; and (2) at least one X must be other than hydrogen when (i) R³ and R⁴ are both hydrogen, (ii) R¹ and R² are selected, independently, from hydrogen and (C₁-C₄)alkyl, and (iii) ring B is mono- or disubstituted with, respectively, one or two halo groups; and
(iii) a compound of the formula XXXI or XXXII, as depicted below, 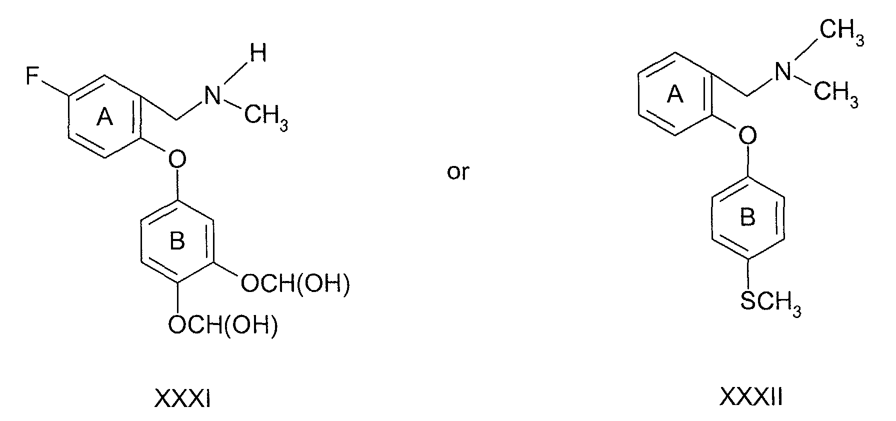 or pharmaceutically acceptable salt thereof.

2. A combination according to claim 1, wherein the compound of formula I, or formula XXX, or pharmaceutically acceptable salt thereof, is selected from the following compounds and their pharmaceutically acceptable salts:
[4-(3,4-Dichlorophenoxy)-biphenyl-3-ylmethyl]-methylamine;
[2-(3,4-Dichlorophenoxy)-5-thiophen-3-ylbenzyl]-methylamine;
[2-(3,4-Dichlorophenoxy)-4-thiophen-3-ylbenzyl]-methylamine;
[2-(3,4-Dichlorophenoxy)-4-furan-2-ylbenzyl]-methylamine;
[2-(3,4-Dichlorophenoxy)-5-furan-2-ylbenzyl]-methylamine;
N-[4'-(3,4-Dichlorphenoxy)-3'-methylaminomethyl-biphenyl-3-yl]-acetamide;
[2-(3,4-Dichlorophenoxy)-5-thiophen-2-ylbenzyl]-methylamine;
[4-(3,4-Dichlorophenoxy)-4'-fluoro-biphenyl-3-ylmethyl]-methyamine;
[2-(3,4-Dichlorophenoxy)-5-[1,2,3]triazol-1-ylbenzyl]-methylamine;
[2-(3,4-Dichlorophenoxy)-5-[1,2,3]triazol-2-ylbenzyl]-methylamine;
[2-(3,4-Dichlorophenoxy)-5-pyridin-2-ylbenzyl]-methylamine;
[2-(3,4-Dichlorophenoxy)-5-pyridin-3-ylbenzyl]-methylamine;
1-[4-(3,4-Dichlorophenoxy)-3-methylaminomethylphenyl]-1H-pyrazol-3-ylamine;
[2-(3,4-Dichlorophenoxy)-5-pyridin-4-ylbenzyl]-methylamine;
[3-(3,4-Dichlorophenoxy)-biphenyl-4-ylmethyl]-methylamine;
[4-(3,4-Dichlorophenoxy)-4'-methyl-biphenyl-3-ylmethyl]-methylamine;
[2-(3,4-Dichlorophenoxy)-4-thiophen-2-ylbenzyl]-methylamine;
[2-(3,4-dichlorophenoxy)-5-thiazol-2-ylbenzyl]-methylamine;
[2-(3,4-dichlorophenoxy)-5-(1H-tetrazol-5-yl)benzyl]-methylamine;
[2-(3,4-dichlorophenoxy)-5-furan-3-ylbenzyl]-methylamine;
{1-[2-(3,4-dichlorophenoxy)-5-[1,2,3]triazol-1 -ylphenyl]ethyl}-methylamine;
{1-[2-(3,4-dichlorophenoxy)-5-[1,2,3]triazol-2-ylphenyl]ethyl}-methylamine;
{1-[2-(3,4-dichlorophenoxy)-5-thiazol-2-ylphenyl]ethyl}-methylamine;
{1-[2-(3,4-dichlorophenoxy)-4-[1,2,4]triazol-1-ylphenyl]ethyl}-methylamine;
[2-(3,4-dichlorophenoxy)-5-(5-methylthiophen-2-yl)benzyl]-methylamine;
[2-(3,4-dichlorophenoxy)-5-[1,2,4]triazol-4-ylbenzyl]-methylamine;
1-[4-(3,4-dichlorophenoxy)-3-(methylaminomethyl)phenyl]-pyrrolidin-2-one;
1-[4-(3,4-dichlorophenoxy)-3-(1-methylaminoethyl)phenyl]-pyrrolidin-2-one;
1-[4-(3,4-dichlorophenoxy)-3-(methylaminomethyl)phenyl]-piperidin-2-one;
[2-(3,4-Dichlorophenoxy)-5-fluorobenzyl]-dimethylamine;
[2-(3,4-Dichlorophenoxy)-5-fluorobenzyl]-methylamine;
[2-(3,4-Dichlorophenoxy)-5-trifluoromethylbenzyl]-dimethylamine;
N-[4-(3,4-Dichlorophenoxy)-3-dimethylaminomethylphenyl]-acetamide;
{1-[2-(3,4-Dichlorophenoxy)phenyl]-ethyl}-dimethylamine;
[2-(3,4-Dichlorophenoxy)-4-trifluoromethylbenzyl]-dimethylamine;
[2-(3,4-Dichlorophenoxy)-4-trifluoromethylbenzyl]-methylam ine;
[4-Chloro-2-(3,4-dichlorophenoxy)-benzyl]-methylamine;
{1-[2-(3,4-Dichlorophenoxy)-5-fluorophenyl]-ethyl}-methylamine;
{1-[2-(3,4-Dichlorophenoxy)phenyl}-ethyl}-methylamine;
{1-[2-(4-Chlorophenoxy)phenyl]ethyl}-methylamine;
[2-(3,4-Dichlorophenoxy)-5-methoxybenzyl]-methylamine;
[2-(4-Chlorophenoxy)-5-fluorobenzyl]-methylamine;
{1-[2-(4-Chlorophenoxy)-5-fluorophenyl]-ethyl}-methylamine;
[2-(3,4-Dichlorophenoxy)-5-methylbenzyl]-dimethylamine;
[4-Bromo-2-(3,4-dichlorophenoxy)-benzyl]-methylamine;
[5-Bromo-2-(3,4-dichlorophenoxy)-benzyl]-methylamine;
[2-(3,4-Dichlorophenoxy)-4,5-dimethoxybenzyl]-methylamine;
[2-(3,4-Dichlorophenoxy)-4-methoxybenzyl]-dimethylamine;
4-(3,4-Dichlorophenoxy)-3-methylaminomethyl-benzonitrile;
[2-(3,4-Dichlorophenoxy)-4,5-dimethylbenzyl]-methylamine;
3-(3,4-Dichlorphenoxy)-4-methylaminomethyl-benzonitrile;
(+)-{1-[2-(3,4-Dichlorophenoxy)-5-fluorophenyl]-ethyl}-methylamine;
(-)-{1-[2-(3,4-Dichlorophenoxy)-5-fluorophenyl]-ethyl}-methylamine;
[2-(3,4-Dichlorophenoxy)-5-trifluoromethyl-benzyl]-methylamine;
[2-(3,4-Dichlorophenoxy)-4-methoxybenzyl]-methylamine;
[2-(4-Chloro-3-fluorophenoxy)-5-fluorobenzyl]-methylamine;
[2-(3-Chloro-4-fluorophenoxy)-5-fluorobenzyl]-methylamine;
(+/-)-2-[2-(3,4-Dichlorophenoxy)-5-fluorophenyl]-pyrrolidine;
(-)-2-[2-(3,4-Dichlorophenoxy)-5-fluorophenyl]-pyrrolidine;
(+)-2-[2-(3,4-Dichlorophenoxy)-5-fluorophenyl]-pyrrolidine; and
2-[2-(3,4-Dichlorophenoxy)-5-fluorophenyl]-N-methylpyrrolidine.

3. A pharmaceutical composition comprising: (a) a dopamine D3 receptor agonist, or a pharmaceutically acceptable salt thereof; (b) a compound selected from the group of formula I, XXX, XXXI or XXXII as defined in claim 1,
or a pharmaceutically acceptable salt thereof; and (c) one or more pharmaceutically acceptable carriers or excipients.

4. A pharmaceutical composition according to claim 3, wherein the compound of formula I or formula XXX, respectively, or pharmaceutically acceptable salt thereof, that is employed in such method is selected from the following compounds and their pharmaceutically acceptable salts:
[4-(3,4-Dichlorophenoxy)-biphenyl-3-ylmethyl]-methylamine;
[2-(3,4-Dichlorophenoxy)-5-thiophen-3-ylbenzyl]-methylamine;
[2-(3,4-Dichlorophenoxy)-4-thiophen-3-ylbenzyl]-methylamine;
[2-(3,4-Dichlorophenoxy)-4-furan-2-ylbenzyl]-methylamine;
[2-(3,4-Dichlorophenoxy)-5-furan-2-ylbenzyl]-methylamine;
N-[4'-(3,4-Dichlorphenoxy)-3'-methylaminomethyl-biphenyl-3-yl]-acetamide;
[2-(3,4-Dichlorophenoxy)-5-thiophen-2-ylbenzyl]-methylamine;
[4-(3,4-Dichlorophenoxy)-4'-fluoro-biphenyl-3-ylmethyl]-methyamine;
[2-(3,4-Dichlorophenoxy)-5-[1,2,3]triazol-1-ylbenzyl]-methylamine;
[2-(3,4-Dichlorophenoxy)-5-[1,2,3]triazol-2-ylbenzyl]-methylamine;
[2-(3,4-Dichlorophenoxy)-5-pyridin-2-ylbenzyl]-methylamine;
[2-(3,4-Dichlorophenoxy)-5-pyridin-3-ylbenzyl]-methylamine;
1-[4-(3,4-Dichlorophenoxy)-3-methylaminomethylphenyl]-1 H-pyrazol-3-ylamine;
[2-(3,4-Dichlorophenoxy)-5-pyridin-4-ylbenzyl]-methylamine;
[3-(3,4-Dichlorophenoxy)-biphenyl-4-ylmethyl]-methylamine;
[4-(3,4-Dichlorophenoxy)-4'-methyl-biphenyl-3-ylmethyl]-methylamine;
[2-(3,4-Dichlorophenoxy)-4-thiophen-2-ylbenzyl]-methylamine;
[2-(3,4-dichlorophenoxy)-5-thiazol-2-ylbenzyl]-methylamine;
[2-(3,4-dichlorophenoxy)-5-(1H-tetrazol-5-yl)benzyl]-methylamine;
[2-(3,4-dichlorophenoxy)-5-furan-3-ylbenzyl]-methylamine;
{1 -[2-(3,4-dichlorophenoxy)-5-[1,2,3]triazol-1 -ylphenyl]ethyl}-methylamine;
{1-[2-(3,4-dichlorophenoxy)-5-[1,2,3]triazol-2-ylphenyl]ethyl}-methylamine;
{1-[2-(3,4-dichlorophenoxy)-5-thiazol-2-ylphenyl]ethyl}-methylamine;
{1-[2-(3,4-dichlorophenoxy)-4-[1,2,4]triazol-1 -ylphenyl]ethyl}-methylamine;
[2-(3,4-dichlorophenoxy)-5-(5-methylthiophen-2-yl)benzyl]-methylamine;
[2-(3,4-dichlorophenoxy)-5-[1,2,4]triazol-4-ylbenzyl]-methylamine;
1-[4-(3,4-dichlorophenoxy)-3-(methylaminomethyl)phenyl]-pyrrolidin-2-one;
1-[4-(3,4-dichlorophenoxy)-3-(1-methylaminoethyl)phenyl]-pyrrolidin-2-one;
1-[4-(3,4-dichlorophenoxy)-3-(methylaminomethyl)phenyl]-piperidin-2-one;
[2-(3,4-Dichlorophenoxy)-5-fluorobenzyl]-dimethylamine;
[2-(3,4-Dichlorophenoxy)-5-fluorobenzyl]-methylamine;
[2-(3,4-Dichlorophenoxy)-5-trifluoromethylbenzyl]-dimethylamine;
N-[4-(3,4-Dichlorophenoxy)-3-dimethylaminomethylphenyl]-acetamide;
{1-[2-(3,4-Dichlorophenoxy)phenyl]-ethyl}-dimethylamine;
[2-(3,4-Dichlorophenoxy)-4-trifluoromethylbenzyl]-dimethylamine;
[2-(3,4-Dichlorophenoxy)-4-trifluoromethylbenzyl]-methylamine;
[4-Chloro-2-(3,4-dichlorophenoxy)-benzyl]-methylamine;
{1-[2-(3,4-Dichlorophenoxy)-5-fluorophenyl]-ethyl}-methylamine;
{1-[2-(3,4-Dichlorophenoxy)phenyl}-ethyl}-methylamine;
{1-[2-(4-Chlorophenoxy)phenyl]ethyl}-methylamine;
[2-(3,4-Dichlorophenoxy)-5-methoxybenzyl]-methylamine;
[2-(4-Chlorophenoxy)-5-fluorobenzyl]-methylamine;
{1-[2-(4-Chlorophenoxy)-5-fluorophenyl]-ethyl}-methylamine;
[2-(3,4-Dichlorophenoxy)-5-methylbenzyl]-dimethylamine;
[4-Bromo-2-(3,4-dichlorophenoxy)-benzyl]-methylamine;
[5-Bromo-2-(3,4-dichlorophenoxy)-benzyl]-methylamine;
[2-(3,4-Dichlorophenoxy)-4,5-dimethoxybenzyl]-methylamine;
[2-(3,4-Dichlorophenoxy)-4-methoxybenzyl]-dimethylamine;
4-(3,4-Dichlorophenoxy)-3-methylaminomethyl-benzonitrile;
[2-(3,4-Dichlorophenoxy)-4,5-dimethylbenzyl]-methylamine;
3-(3,4-Dichlorphenoxy)-4-methylaminomethyl-benzonitrile;
(+)-{1-[2-(3,4-Dichlorophenoxy)-5-fluorophenyl]-ethyl}-methylamine;
(-)-{1-[2-(3,4-Dichlorophenoxy)-5-fluorophenyl]-ethyl}-methylamine;
[2-(3,4-Dichlorophenoxy)-5-trifluoromethyl-benzyl]-methylamine;
[2-(3,4-Dichlorophenoxy)-4-methoxybenzyl]-methylamine;
[2-(4-Chloro-3-fluorophenoxy)-5-fluorobenzyl]-methylamine;
[2-(3-Chloro-4-fluorophenoxy)-5-fluorobenzyl]-methylamine;
(+/-)-2-[2-(3,4-Dichlorophenoxy)-5-fluorophenyl]-pyrrolidine;
(-)-2-[2-(3,4-Dichlorophenoxy)-5-fluorophenyl]-pyrrolidine;
(+)-2-[2-(3,4-Dichlorophenoxy)-5-fluorophenyl]-pyrrolidine; and
2-[2-(3,4-Dichlorophenoxy)-5-fluorophenyl]-N-methylpyrrolidine.

5. A pharmaceutical composition according to claim 3 or 4, wherein the amount of the dopamine D3 receptor agonist, or pharmaceutically acceptable salt thereof, in said composition is from about 0.05 mg to about 1500 mg and the amount of the compound of formula I or formula XXX or formula XXXI or XXXII, respectively, or pharmaceutically acceptable salt thereof, is from about 0.7 mg to about 700 mg.

6. A pharmaceutical composition according to claim 5, wherein the amount of the dopamine D3 receptor agonist, or pharmaceutically acceptable salt thereof, in said composition is from about 1 mg to about 500 mg and the amount of the compound of formula I or formula XXX or formula XXXI or XXXII, respectively, or pharmaceutically acceptable salt thereof, is from about 1 mg to about 500 mg.

7. Use of a compound of formula I or formula XXX or formula XXXI or XXXII, according to claim 1, for the manufacture of a medicament in combination with a dopamine D3 receptor agonist for the curative, palliative or prophylactic treatment of depression or anxiety in a mammal.

8. Use according to claim 7, wherein the dopamine D3 receptor agonist, or pharmaceutically acceptable salt thereof, and the compound of formula I or formula XXX or formula XXXI or XXXII, respectively, or pharmaceutically acceptable salt thereof, are administered as part of the same dosage form.

9. Use according to claim 7 or claim 8, wherein the compound of formula I or formula XXX or formula XXXI or XXXII, respectively, or pharmaceutically acceptable salt thereof, is administered in an amount ranging from about 0.7 mg per day to about 700 mg per day, and the dopamine D3 receptor agonist, or pharmaceutically acceptable salt thereof, is administered in an amount ranging from about 0.05 mg per day to about 1500 mg per day.

10. Use according to claim 9, wherein the compound of formula I or formula XXX or formula XXXI or XXXII, respectively, or pharmaceutically acceptable salt thereof, is administered in an amount ranging from about 1 mg per day to about 500 mg per day.
